(19) Europäisches Patentamt
European Patent Office
Office européen des brevets

(11) Veröffentlichungsnummer: **0 115 469**
**B1**

(12) # EUROPÄISCHE PATENTSCHRIFT

(45) Veröffentlichungstag der Patentschrift: **20.05.87**

(21) Anmeldenummer: **84810002.0**

(22) Anmeldetag: **04.01.84**

(51) Int. Cl.⁴: **C 07 D 471/14,** C 07 D 495/14, A 61 K 31/435 // C07D213/74, C07D231/34, C07D471/04, C07D401/12, C07D213/80, C07D213/75, C07D401/04 ,(C07D471/14, 231:00, 221:00, 221:00), (C07D471/14, 235:00, 231:00, 221:00),(C07D495/14, 333:00, 231:00, 221:00)

(54) **Heterocyclisch ankondensierte Pyrazolo(3,4-d)pyridin-3-one.**

(30) Priorität: **10.01.83 US 457105**

(43) Veröffentlichungstag der Anmeldung: **08.08.84 Patentblatt 84/32**

(45) Bekanntmachung des Hinweises auf die Patenterteilung: **20.05.87 Patentblatt 87/21**

(84) Benannte Vertragsstaaten: **AT BE CH DE FR GB IT LI LU NL SE**

(56) Entgegenhaltungen:
**EP-A-0 022 078**
**FR-A-2 102 217**
**FR-A-2 116 469**

**CHEMICAL ABSTRACTS, Band 97, Nr. 19, 8. November 1982, Seite 706, Nr. 162862n, Columbus, Ohio, US, R.M. TITKOVA et al.: "Synthesis of 4-substituted 3-carbethoxy(carboxy)-1,5-naphthyridines, their properties and biological activity"**

**Die Akte enthält technische Angaben, die nach dem Eingang der Anmeldung eingereicht wurden und die nicht in dieser Patentschrift enthalten sind.**

(73) Patentinhaber: **CIBA- GEIGY AG, Klybeckstrasse 141, CH- 4002 Basel (CH)**

(72) Erfinder: **Yokoyama, Naokata, Dr., 200 Winston Drive Apt. 2103, Cliffside Park New Jersey 07010 (US)**

**EP 0 115 469 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

In der EP-A-22 078 sind 2-Aryl-pyrazolo[4,3-c]chinolin-3-on-Verbindungen mit anxiolytischen und antidepressiven Eigenschaften beschrieben worden.

Aus der FR-A-2 102 217 sind 5-Methyl-dipyrazolo[3,4-b:3,4'-d]-pyridin-3(2H)-on-Verbindungen mit antidepressiven und anxiolytischen Wirkungen bekannt.

Aus der FR-A-2 166 469 sind Dipyrazolo [3,4-b:3,4'-d]-pyridin-3-on-Verbindungen mit anxiolytischen und antidepressiven Wirkungen bekannt.

Aus den Chemical Abstracts, Band 97, Nr. 19, vom 8. November 1982, Seite 706, Nr. 162 862 n, Columbus, Ohio, US, R.M. Titkova et al. "Synthesis of 3-carbethoxy(carboxy)-1,5-naphthyridines, their properties and biological activity" sind Verbindungen der Formeln

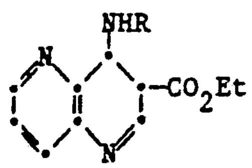 (II) 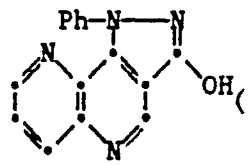 (III) 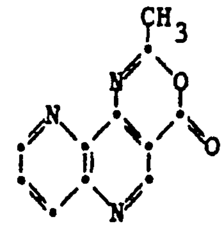 (IV)

mit antimikrobiellen Eigenschaften bekannt.

Der vorliegenden Erfindung liegt die Aufgabe zugrunde, neue Arzneistoffe und -mittel mit Wirkung auf das Nervensystem zu schaffen, das Benzodiazepin-Rezeptoren modulierende Eigenschaften besitzt.

Die Aufgabe wird erfindungsgemäss durch die Bereitstellung der neuen Pyrazolo-pyridin-3-on-Verbindungen der Formel IA bzw. IB gelöst.

Die Erfindung betrifft neue 2-substituierte-[b]-heterocyclisch ankondensierte Pyrazolo[3,4-d]-pyridin-3-one der Formeln IA oder IB

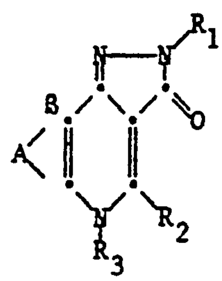 (IA) oder 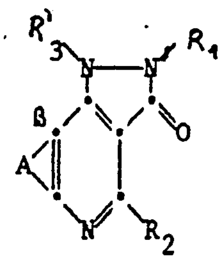 (IB)

worin A zusammen mit den 2 Kohlenstoffatomen, an die es gebunden ist, einen ankondensierten ungesättigten heterocyclischen Ring darstellt aus der Gruppe (a) Thieno, Furo und gegebenenfalls N-Niederalkyl substituiertes Pyrrolo, wobei die Kohlenstoffatome in jedem dieser Ringe unsubstituiert oder eines von ihnen durch Niederalkyl, Carbo-(nieder)-alkoxy, Halogen oder Trifluormethyl substituiert ist; (b) gegebenenfalls N-Niederalkyl substituiertes Imidazo, worin das Kohlenstoffatom im genannten Ring gegebenenfalls durch Niederalkyl, Niederalkoxy, Halogen oder Trifluormethyl substituiert ist; (c) Thiazolo, Oxazolo, Isoxazolo, 2,3-, 3,4- oder 4,3-Pyrido und Pyridazino, wobei die Kohlenstoffatome, die diese Ringe bilden, unsubstituiert sind oder eines oder zwei davon durch Niederalkyl, Niederalkoxy oder Halogen substituiert sind; (d) Pyrimido und Pyrazino, wobei die Kohlenstoffatome in diesen Ringen unsubstituiert oder eines davon durch Niederalkyl oder Niederalkoxy substituiert ist; $R_1$ gegebenenfalls durch Niederalkyl, Niederalkoxy, Halogen oder Trifluoromethyl mono- oder disubstituiertes Phenyl bedeutet; oder $R_1$ einen aromatischen heterocyclischen Rest aus der Gruppe Pyridyl, Chinolyl, Isochinolyl, Pyrinidyl und Thiazolyl oder jeden der genannten heterocyclischen Reste bedeutet, die durch Niederalkyl, Niederalkoxy oder Halogen mono- oder disubstituiert sind; und $R_2$, $R_3$ und $R'_3$ unabhängig voneinander Wasserstoff oder Niederalkyl bedeuten; oder Salze davon, insbesondere pharmazeutisch annehmbare Salze; Verfahren für ihre Herstellung, pharmazeutische Zubereitungen, die diese Verbindungen enthalten und ihre therapeutische Anwendung.

Die genannten Verbindungen der Formel IA oder IB sind also $R_1$-substituierte (Thieno, Furo, Pyrrolo, laidazo, Thiazolo, Oxazolo, Isoxazolo, Pyrido, Pyridazino, Pyrimido oder Pyrazino)-pyrazolo-[2,3-d]-pyridin-3-one, wie oben definiert.

Besonders bevorzugte Verbindungen sind die genannten Verbindungen der Formel IA oder IB, worin A die oben genannte Bedeutung hat; $R_2$, $R_3$ und $R'_3$ Wasserstoff bedeuten und

a) worin $R_1$ gegebenenfalls durch Niederalkyl, Niederalkoxy oder Halogen monosubstituiertes Phenyl bedeutet;

b) worin $R_1$ 2-Pyridyl, 5-(Methyl, Methoxy oder Chlor)-2-pyridyl, 3-Pyridyl, 6-(Methyl oder Methoxy)-3-pyridyl oder 4-Pyridyl bedeutet;

c) worin $R_1$ 3-Pyrimidyl, 5-(Methyl, Methoxy oder Chlor)-2-pyrimidyl, 4-Pyrimidyl oder 5-Pyrimidyl bedeutet;
d) worin $R_1$ Thiazolyl oder 5-(Methyl, Methoxy oder Chlor)-2-thiazolyl bedeutet;
e) worin $R_1$ 2-Chinolyl, 3-Chinolyl oder 7-Chlor-4-chinolyl bedeutet; und
f) worin $R_1$ 1-Isochinolyl bedeutet;
Tautomere davon; oder Salze, insbesondere pharmazeutisch verwendbare Salze davon.

Eine besondere Ausführungsform der Erfindung betrifft Pyrido-pyrazo-lo[3,4-d]pyridin-3-one (insbesondere Pyrazolo[4,3-c][1,6],[1,7] oder [1,8]-naphthyridin-3-one) und wird dargestellt durch Verbindungen der Formel IA oder IB, worin A zusammen mit den beiden Kohlenstoffatomen, an welche es gebunden ist, ankondensierte 2,3-3,4- oder 4,3-Pyrido (vorzugsweise 3,4-oder 4,3-pyrido) oder jeden der genannten Pyridoringe darstellt, die durch Niederalkyl, Niederalkoxy oder Halogen mono- oder disubstituiert sind; und $R_1$, $R_2$, $R_3$ und $R'_3$ die oben angegebenen Bedeutungen haben; oder Salze davon, insbesondere pharmazeutisch verwendbare Salze.

Eine andere Ausführungsform der Erfindung betrifft Thieno-pyrazolo-[3,4-d]pyridin-3-one und wird dargestellt durch die Verbindungen der Formel IA und IB, worin A zusammen mit den 2 Kohlenstoffatomen, an welche es gebunden ist, ankondensiertes 2,3-oder 3,2-Thieno darstellt und wobei die Kohlenstoffatome im genannten Thienoring unsubstituiert sind oder einer davon durch Niederalkyl, Carbo(nieder)-alkoxy, Halogen oder Trifluoromethyl substituiert ist, und $R_1$, $R_2$, $R_3$ und $R'_3$ die oben angegebene Bedeutung haben; oder Salze davon, insbesondere pharmazeutisch verwendbare Salze.

Eine weitere Ausführungsform der Erfindung betrifft Imidazo-pyrazo-lo[3,4-d]pyridin-3-one und wird dargestellt durch Verbindungen der Formeln IA oder IB, worin A zusammen mit den 2 Kohlenstoffatomen, an welche es gebunden ist, gegebenenfalls N-Niederalkyl substituiertes ankondensiertes 5,4-Imidazo darstellt, und die Kohlenstoffatome im genannten 5,4-Imidazoring gegebenenfalls durch Niederalkyl, Phenyl oder durch Niederalkyl, Niederalkoxy, Halogen oder Trifluormethyl monosubstituiertes Phenyl substituiert ist, und $R_1$, $R_2$, $R_3$ und $R'_3$ die oben angegebene Bedeutung haben; oder Salze davon, insbesondere pharmazeutisch anwendbare Salze.

Besonders bevorzugte Verbindungen sind die genannten Pyrazolo-[4,3-c][1,6]naphthyridin-3(5H)-one der Formel II

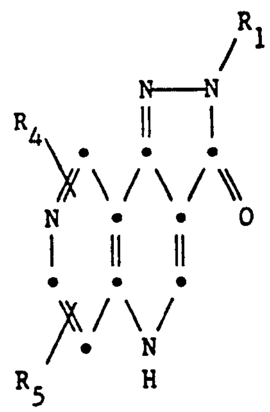

(II),

worin $R_1$ gegebenenfalls durch Niederalkyl, Niederalkoxy, Halogen oder Trifluoromethyl mono- oder disubstituiertes Phenyl darstellt; oder $R_1$ einen aromatischen heterocyclischen Rest aus der Gruppe Pyridyl, Chinolyl, Isochinolyl, Pyrimidyl oder Thiazolyl darstellt, oder einen dieser genannten Reste, der durch Niederalkyl, Niederalkoxy oder Halogen mono- oder disubstituiert ist; $R_4$ und $R_5$ unabhängig voneinander Wasserstoff, Niederalkyl, Niederalkoxy oder Halogen darstellen; oder Tautomere davon; oder Salze davon, insbesondere pharmazeutisch anwendbare Salze.

Besonders bevorzugt sind Verbindungen der Formel II

a) worin $R_1$ gegebenenfalls durch Niederalkyl, Niederalkoxy oder Halogen monosubstituiertes Phenyl ist, $R_4$ Wasserstoff, Methoxy, Methyl, Fluor oder Chlor ist, $R_5$ Wasserstoff ist; oder Tautomere davon; oder Salze davon, insbesondere pharmazeutisch anwendbare Salze;

b) worin $R_1$ 2-Pyridyl, 5-(Methyl, Methoxy oder Chlor)-2-pyridyl, 3-Pyridyl, 6-(Methyl oder Methoxy)-3-pyridyl oder 4-Pyridyl ist; $R_4$ Wasserstoff, Methyl, Methoxy, Fluoro oder Chlor ist; $R_5$ Wasserstoff ist; oder Tautomere davon; oder Salze davon, insbesondere pharmazeutisch verwendbare Salze.

c) worin $R_1$ 2-Pyrimidyl, 5-(Methyl, Methoxy oder Chlor)-2-pyrimidyl 4-Pyrimidyl oder 5-Pyrimidyl ist; $R_4$ Wasserstoff, Methoxy, Methyl, Fluor oder Chlor ist; $R_5$ Wasserstoff ist; oder Tautomere davon; oder Salze davon, insbesondere pharmazeutisch verwendbare Salze;

d) worin $R_1$ 2-Thiazolyl oder 5-(Methyl, Methoxy oder Chlor)-2-thiazolyl ist; $R_4$ Wasserstoff, Methoxy, Methyl, Fluor oder Chlor ist; $R_5$ Wasserstoff ist; oder Tautomere davon; oder Salze davon, insbesondere pharmazeutisch verwendbare Salze;

e) worin $R_1$ 2-Chinolyl, 3-Chinolyl oder 7-Chlor-4-chinolyl ist; $R_4$ Wasserstoff, Methyl, Fluor oder Chlor ist; $R_5$ Wasserstoff ist; oder Tautomere davon, oder Salze davon, insbesondere pharmazeutisch verwendbare Salze; und

f) worin $R_1$ 1-Isochinolyl ist; $R_4$ Wasserstoff, Methoxy, Methyl, Fluor oder Chlor ist; $R_5$ Wasserstoff ist; oder

Tautomere davon; oder Salze davon, insbesondere pharmazeutisch verwendbare Salze.

Ebenfalls bevorzugt sind Pyrazolo[4,3-c][1,7]-naphthyridin-3-one der Formel III

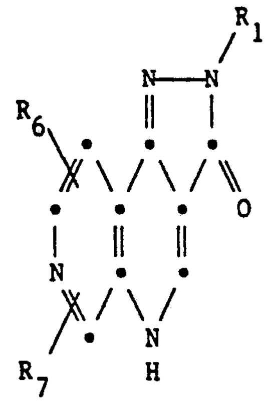

(III),

worin $R_1$ gegebenenfalls durch Niederalkyl, Niederalkoxy, Halogen oder Trifluoromethyl mono- oder disubstituiertes Phenyl bedeutet; oder $R_1$ einen aromatischen heterocyclischen Rest aus der Gruppe Pyridyl, Chinolyl, Isochinolyl, Pyrimidyl und Thiazolyl bedeutet, oder einen der genannten Reste, der durch Niederalkyl, Niederalkoxy oder Halogen mono- oder disubstituiert ist; $R_6$ und $R_7$ unabhängig voneinander Wasserstoff, Niederalkyl, Niederalkoxy oder Halogen bedeuten; oder Tautomere davon; oder Salze davon, insbesondere pharmazeutisch verwendbare Salze davon.

Ganz besonders bevorzugt sind Verbindungen der Formel III

a) worin $R_1$ gegebenenfalls durch Niederalkyl, Niederalkoxy oder Halogen monosubstituiertes Phenyl bedeutet; $R_6$ Wasserstoff, Methoxy, Methyl, Fluor oder Chlor bedeutet; $R_7$ Wasserstoff bedeutet; oder Tautomere davon; oder Salze davon, insbesondere pharmazeutisch verwendbare Salze davon;

b) worin $R_1$ 2-Pyridyl, 5-(Methyl, Methoxy oder Chlor)-2-pyridyl, 3-Pyridyl, 6-(Methyl oder Methoxy)-3-pyridyl oder 4-Pyridyl bedeutet; $R_6$ Wasserstoff, Methyl, Methoxy, Fluor oder Chlor bedeutet; $R_7$ Wasserstoff bedeutet, oder Tautomere davon, oder Salze davon, insbesondere pharmazeutisch verwendbare Salz;

c) worin $R_1$ 2-Pyrimidyl, 5-(Methyl, Methoxy oder Chlor)-2-pyrimidyl, 4-Pyrimidyl oder 5-Pyrimidyl bedeutet, $R_6$ Wasserstoff, Methoxy, Methyl, Fluor oder Chlor bedeutet; $R_7$ Wasserstoff bedeutet; oder Tautomere davon; oder Salze davon, insbesondere pharmazeutisch verwendbare Salze; und

d) worin $R_1$ 2-Thiazolyl oder 5-(Methyl, Methoxy oder Chlor)-2-thiazolyl bedeutet; $R_6$ Wasserstoff, Methoxy, Methyl, Fluor oder Chlor bedeutet; $R_7$ Wasserstoff bedeutet; oder Tautomere davon; oder Salze davon, insbesondere pharmazeutisch verwendbare Salze.

Ebenfalls bevorzugt sind die Thieno[2,3-b]pyrazolo[3,4-d]-pyridin-3-one der Formel IV

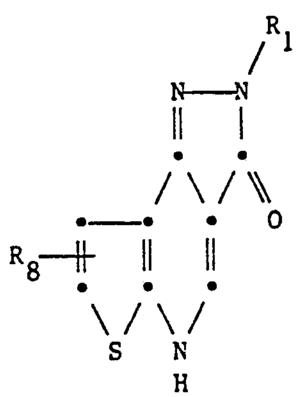

(IV),

worin $R_1$ gegebenenfalls durch Niederalkyl, Niederalkoxy, Halogen oder Trifluoromethyl mono- oder disubstituiertes Phenyl bedeutet, oder einen aromatischen heterocyclischen Rest aus der Gruppe Pyridyl, Chinolyl, Isochinolyl, Pyrimidyl und Thiazolyl, oder einen der genannten heterocyclischen Reste, der durch Niederalkyl, Niederalkoxy oder Halogen mono- oder disubstituiert ist; $R_8$ Wasserstoff, Niederalkyl, Carbo-(nieder)-alkoxy, Halogen oder Trifluoromethyl bedeutet; oder Tautomere davon, oder Salze davon, insbesondere pharmazeutisch verwendbare Salze.

Besonders bevorzugt sind Verbindungen der Formel IV

a) worin $R_1$ gegebenenfalls durch Niederalkyl, Niederalkoxy oder Halogen mono-substituiertes Phenyl ist; oder Tautomere davon, oder Salze davon, insbesondere pharmazeutisch verwendbare Salze;

b) worin $R_1$ 2-Pyridyl, 5-(Methyl, Methoxy oder Chlor)-2-pyridyl, 3-Pyridyl, 6-(Methyl oder Methoxy)-3-pyridyl oder 4-Pyridyl ist; $R_8$ Wasserstoff oder Halogen ist; oder Tautomere davon; oder Salze davon, insbesondere

pharmazeutisch verwendbare Salze; und

c) worin $R_1$ 2-Thiazolyl oder 5-(Methyl, Methoxy oder Chlor)-2-thiazolyl ist; $R_8$ Wasserstoff oder Halogen ist; oder Tautomere davon; oder Salze davon, insbesondere pharmazeutisch verwendbare Salze davon.

Ebenfalls bevorzugt sind Thieno[3,2-b]pyrazolo[3,4-d]pyridin-3-one der Formel V

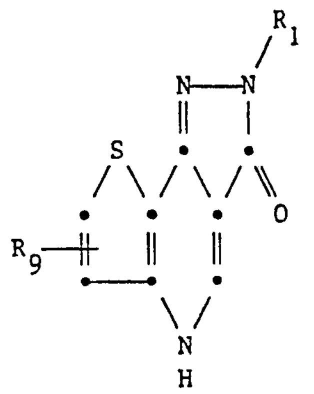

(V),

worin $R_1$ gegebenenfalls durch Niederalkyl, Niederalkoxy, Halogen oder Trifluoromethyl mono- oder disubstituiertes Phenyl bedeutet; oder einen aromatischen heterocyclischen Rest aus der Gruppe Pyridyl, Chinolyl, Isochinolyl, Pyrimidyl oder Thiazolyl oder einen der genannten heterocyclischen Reste, der durch Niederalkyl, Niederalkoxy oder Halogen mono- oder disubstituiert sind; $R_9$ Wasserstoff, Niederalkyl, Carbo-(nieder)-alkoxy, Halogen oder Trifluoromethyl bedeutet; oder Tautomere davon; oder Salze davon, insbesondere pharmazeutisch verwendbare Salze.

Besonders bevorzugt sind Verbindungen der Formel V

a) worin $R_1$ gegebenenfalls durch Niederalkyl, Niederalkoxy oder Halogen monosubstituiertes Phenyl ist; $R_9$ Wasserstoff oder Halogen ist; oder Tautomere davon; oder Salze davon, insbesondere pharmazeutisch verwendbare Salze; und

b) worin $R_1$ 2-Pyridyl, 5-(Methyl, Methoxy oder Chlor)-2-pyridyl, 3-Pyridyl, 6-(Methyl oder Methoxy)-3-pyridyl oder 4-Pyridyl ist; $R_9$ Wasserstoff oder Halogen ist; oder Tautomere davon; oder Salze davon, insbesondere pharmazeutisch verwendbare Salze.

Ebenfalls bevorzugt sind die Imidazo[4,5-b]pyrazolo[3,4-d]-pyridin-3-one der Formel VI

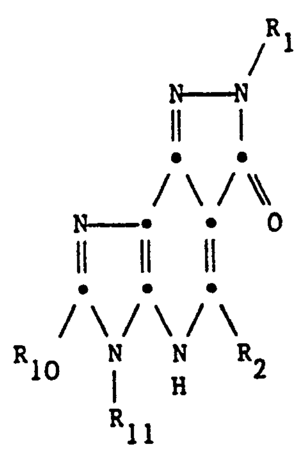

(VI),

worin $R_1$ gegebenenfalls durch Niederalkyl, Niederalkoxy, Halogen oder Trifluoromethyl mono- oder disubstituiertes Phenyl bedeutet; oder ein aromatischer heterocyclischer Rest aus der Gruppe Pyridyl, Chinolyl, Isochinolyl, Pyrimidyl und Thiazolyl oder einer der genannten heterocyclischen Reste, der durch Niederalkyl, Niederalkoxy oder Halogen substituiert, ist; $R_2$ Wasserstoff oder Niederalkyl bedeutet; $R_{10}$ Wasserstoff, Niederalkyl oder gegebenenfalls durch Niederalkyl, Niederalkoxy, Halogen oder Trifluoromethyl monosubstituiertes Phenyl bedeutet; $R_{11}$ Wasserstoff oder Niederalkyl bedeutet; oder Tautomere davon; oder Salze davon, insbesondere pharmazeutisch verwendbare Salze davon.

Besonders bevorzugt sind Verbindungen der Formel VI

a) worin $R_1$ gegebenenfalls durch Niederalkyl, Niederalkoxy oder Halogen monosubstituiertes Phenyl ist; $R_2$, $R_{10}$ und $R_{11}$ unabhängig voneinander Wasserstoff oder Methyl sind; oder Tautomere davon, oder Salze davon, insbesondere pharmazeutisch verwendbare Salze; und

b) worin $R_1$ 2-Pyridyl, 5-(Methyl, Methoxy oder Chlor)-2-pyridyl, 3-Pyridyl, 6-(Methyl oder Methoxy)-4-pyridyl; $R_2$, $R_{10}$ und $R_{11}$ unabhängig voneinander Wasserstoff oder Methyl sind, oder Tautomere davon; oder Salze

davon, insbesondere pharmazeutisch annehmbare Salze.

Die verwendeten allgemeinen Definitionen haben im Rahmen der vorliegenden Erfindung die folgenden Bedeutungen.

Der Ausdruck "nieder" definiert in den oben oder nachfolgend genannten organischen Resten oder Verbindungen solche mit höchstens 7, vorzugsweise 4, insbesondere 1 oder 2, Kohlenstoffatomen.

Halogen ist vorzugsweise Fluor oder Chlor, es kann aber auch Brom oder Jod sein.

Eine Niederalkylgruppe oder eine solche in einer Niederalkoxygruppe oder in einer anderen alkylierten Gruppe ist in erster Linie Methyl, aber auch Ethyl, n- oder i-(Propyl, Butyl, Pentyl, Hexyl oder Heptyl), z.B. 2-Methyl-propyl oder 3-Methyl-butyl. Carbo-(nieder)alkoxy stellt Niederalkoxycarbonyl, z.B. Ethoxy- oder Methoxycarbonyl dar.

Pyridyl ist 2-, 3- oder 4-Pyridyl, vorzugsweise 3-Pyridyl.

Chinolyl ist vorzugsweise 2-, 3- oder 4-Chinolyl, insbesondere 3-Chinolyl.

Isochinolyl ist vorzugsweise 1-, 3- oder 4-Isochinolyl, im besonderen 1-Isochinolyl.

Pyrimidyl ist 2-, 4- oder 5-Pyrimidyl, vorzugsweise 2- oder 5-Pyrimidyl.

Thiazolyl ist vorzugsweise 2-Thiazolyl.

Die Verbindungen der Erfindung, in welchen $R_3$ und $R'_3$ Wasserstoff bedeuten, können durch eine der tautomeren Strukturformeln IA oder IB, vorzugsweise durch die Struktur IA, dargestellt werden. Weiter können die genannten 3-Oxoverbindungen, unter gewissen Bedingungen, auch als 3-Hydroxy-Tautomeren vorhanden sein. Alle diese Tautomeren sind innerhalb des Umfangs der vorliegenden Erfindung. Die genannten Verbindungen bilden, insbesondere in der Form von 3-Hydroxy-Verbindungen, Salze mit starken Basen. Die Salze sind vorzugsweise Alkalimetall-, z.B. Natrium- oder Kaliumsalze von 1- oder 5-unsubstituierten Verbindungen ($R_3$ und $R'_3$ = H).

Weiter können die Verbindungen der Formel IA oder IB Säureadditionssalze bilden, welche vorzugsweise solche von pharmazeutisch verwendbaren anorganischen oder organischen Säuren sind. Solche Säuren sind z.B. starke Mineralsäuren, wie Halogenwasserstoffsäure, z.B. Chlorwasserstoff- oder Bromwasserstoffsäure, oder Schwefel-, Phosphor-oder Salpetersäure; aliphatische oder aromatische Carbon- oder Sulfonsäuren, z.B. Essig-, Propion-, Bernstein-, Glykol-, Milch-, Apfel-, Wein-, Glucon-, Zitronen-, Malein-, Fumar-, Hydroxymalein-, Brenztrauben-, Phenylessig-, Benzoe-, 4-Aminobenzoe-, Anthranil-, 4-Hydroxybenzoe-, Salicyl-, Aminosalicyl-, Pamoe-, Nikotin-, Methansulfon-, Ethansulfon-, Hydroxyethansulfon-, Benzolsulfon-, p-Toluolsulfon-, Naphthalinsulfon-, Sulfanyl-, Cyclohexylsulfaminsäure; oder die Ascorbinsäure.

Die Verbindungen der Erfindung zeigen wertvolle pharmakologische Eigenschaften, z.B. Nervensystem regulierende Effekte, welche unter anderem durch Beeinflussung der Benzodiazepin-Rezeptor-Aktivität in Säugern zustandekommen. Die Verbindungen sind somit nützlich für die Behandlung von Krankheiten, des Nervensystems z.B. solchen, welche auf die Beeinflussung von Benzodiazepin-Rezeptoren ansprechen.

Die Verbindungen der Erfindung binden sich an den Benzodiazepin-Rezeptor und zeigen anxiolytische und/oder antikonvulsive Effekte, oder sie weisen Antagonismus gegen die Wirkungen von Benzodiazepin-Medikamenten auf. Diese Effekte können durch in vitro oder in-vivo Testmethoden, vorzugsweise an Säugetieren, z.B. Mäusen, Ratten oder Affen, als Testobjekte, nachgewiesen werden. Die genannten Verbindungen können ihnen enteral oder parenteral, vorzugsweise oral, oder subkutan, intravenös oder intraperitoneal, z.B. durch Steckkapseln oder in Form von Stärke enthaltenden Suspensionen oder wässerigen Lösungen oder Suspensionen, verabreicht werden. Die verwendete Dosis kann in einem Bereich von ungefähr zwischen 0,1 und 100 mg/kg/Tag, vorzugsweise ungefähr 0,1 und 50 mg/kg/Tag, insbesondere zwischen 1 und 30 mg/kg/Tag liegen.

Die Benzodiazepin-Rezeptor-bindenden Eigenschaften weisen auf die Nervensystem-regulierende Wirkung der genannten neuen Verbindungen hin. Diese Eigenschaften werden im in-vitro-Test für die Rezeptor-Bindung, z.B. wie in Nature 266, 732 (1977) oder Proc. Nat. Acad. Sci. USA 74, 3805 (1977) beschrieben, bestimmt. Das Diazepam bindet sich spezifisch und mit hoher Affinität an rohe synaptosomale Membranpräparate aus dem Vorderhirn der Ratte. Diese Bindung wird durch andere anxiolytische Verbindungen gehemmt. Verwendet man mit Tritium markiertes Diazepam, so lässt sich die Wechselwirkung von anderen Wirkstoffen mit dem genannten Rezeptor wie folgt leicht beurteilen: Membranen aus dem Vorderhirn der Ratte werden bei 0-5° 30 Minuten mit 2 nM Tritium markiertem Diazepam und mit verschiedenen Konzentrationen der getesteten Substanzen in einem Puffer-Medium, bei einem pH-Wert von 7,5, inkubiert. Lösungen, welche die getesteten Substanzen in verschiedenen Konzentrationen enthalten, werden durch Verdünnen einer 4,2 mM Stammlösung in Dimethylacetamid-Aethanol (1:10) mit 50 mM Tris-HCl-Puffer vom pH-Wert 7,5 hergestellt. Die Membranen, welche die Rezeptoren mit verschiedenen Mengen des mit Tritium markierten Diazepams enthalten, werden auf Glasfaserfiltern filtriert. Diese werden dann in einem Flüssigkeits-Szintillations-Zähler analysiert. Die Konzentration der erfindungsgemässen Verbindungen, welche zur 50%igen Hemmung der spezifischen Bindung von 2 nM mit Tritium markiertem Diazepam notwendig ist, d.h. die $IC_{50}$ (Inhibitory Concentration) lässt sich graphisch berechnen.

Die Benzodiazepin-Rezeptor bindende Wirkung wird in vivo im wesentlichen wie in Eur. J. Pharmacol. 48, 213 (1978) und Nature 275, 551 (1978) beschrieben, ermittelt.

Die Testverbindungen werden in einem Maisstärke-Trägermaterial, peroral oder intraperoral, an Mäuse oder Ratten verabreicht. Nach 30 Minuten injiziert man in die Schwanzvene 3H-Flunitrazepan (2 nMol/kg in physiologischer Salzlösung). Zwanzig Minuten nach der Verabreichung des Flunitrazepans werden die Tiere getötet. Ihr Gehirn wird dann, durch Bestimmung der Radioaktivität in einem Flüssigkeits-Scintillations-Zähler,

auf Bindung des Radioligands zum Rezeptor untersucht. Die Abnahme der Bindung des 3H-Flunitrazepams in den mit dem Wirkstoff behandelten Tieren (im Vergleich zu der Bindung, die in den lediglich mit dem Trägermaterial behandelten Tieren festgestellt ist) weist auf die Benzodiazepin-Rezeptor-bindende Wirkung der getesteten Verbindung hin.

Die anxiolytischen Effekte werden z.B. gemäss dem Cook-Davidson-Konflikt-Verfahren, in welchem man männliche Wistar-Ratten verwendet, festgestellt. Diese Ratten werden durch Diät, aber keine Einschränkung der Wasserzufuhr, bei 80% des normalen Körpergewichts gehalten. Sie werden trainiert, einen Hebel innerhalb einer Konditionierungskammer zu drücken. Die Kammer enthält auch einen Tropfer für Flüssigkeit, eine Lichtquelle, einen Lautsprecher und einen Gitterboden. Sowohl der Hebel als auch das Gitter sind an eine Elektroschock-Quelle angeschlossen. Die Kammer befindet sich in einem schallgedämpften Raum, in welchem im Laufe des Testverfahrens eine weisse Geräuschquelle die auswärtigen Höreinflüsse abschirmt. Jede Testperiode von 47 Minuten besteht aus zwei alternierenden Programmen. Das erste ist ein variables Intervall (VI)-Programm von 30 Sekunden, das sich während 5 Minuten wiederholt. In diesem Programm wird 30 Sekunden nach der ersten Hebelbetätigung eine gesüsste Kondensmilchration der Ratte abgegeben. Die vom Wirkstoff ausgelöste Abnahme dieser Leistung der Hebelbetätigung wird als ein Hinweis auf das neurologische Defizit betrachtet. Unmittelbar nach dem VI-Programm werden gleichzeitig ein Ton von 1000 Hz und ein Lichtsignal aktiviert, welche den Anfang des zweiten, sogenannten Fixed Ratio (FR)-Programmes anzeigen, welches 2 Minuten dauert. In diesem Zeitintervall wird unmittelbar nach dem zehnten Hebeldruck, gleichzeitig mit der Milchration, ein elektrischer Fuss-Schock verabreicht, wodurch eine Konfliktsituation entsteht. Die Intensität des genannten Schocks liegt zwischen 2,0 und 3,6 mA. Sie variiert für jedes Versuchstier, um es während der ganzen Sitzungsperiode auf ungefähr 25 bis 100 Hebeldrücke inerhalb dieses Programms einzustellen. Die vom Wirkstoff hervorgerufene Steigerung dieser Leistung während des FR-Programms gilt als Hinweis auf die anxiolytische Wirkung. Diese gesteigerte Leistung wird an der erhöhten Anzahl der während sechs FR-Testperioden erlittenen Fuss-Schocks bestimmt. Jede Testperiode dauert 2 Minuten.

Die antikonvulsiven Effekte werden z.B. gemäss der Standard-Metrazol- (Pentylentetrazol-) und der maximalen Elektroschock-Testmethode für die Beurteilung der antikonvulsiven Aktivität, z.B. nach oraler Verabreichung an die Ratte wahrgenommen.

Männliche Wistar-Ratten (130-175 g) werden vor dem Testen 18 Stunden fasten gelassen, wobei sie jedoch normalen Zugang zu Wasser haben. Die geprüfte Verbindung wird mittels eines Maisstärke-Trägermaterials durch orale Intubation eines Volumens von 10 ml/kg pro Körpergewicht verabreicht. Eine Stunde nach der Verabreichung der Testverbindungen erhalten die Tiere intravenös (in die kaudale Vene) eine Dosis von 24 mg/kg Metrazol in Wasser, in einem Volumen von 2,5 ml/kg pro Körpergewicht. Die Ratten werden dann gleich in Kunststoff-Zylinder gestellt und während der nächsten 60 Sekunden, mindestens 5 Sekunden, zwecks Feststellung von klonischen Anfällen beobachtet. Der $ED_{50}$-Wert ist die Dosis, bei welcher die Hälfte der Tiere vor den durch Metrazol hervorgerufenen Krampf-Anfällen während der Beobachtungsperioden geschützt wird.

Der Benzodiazepin-Antagonismus wird mit Hilfe vom Antaganonismus gegenüber der antikonvulsiven Wirkung vom Diazepam im Metrazol-Modell gemessen. Das Diazepam (5,4 mg/kg/p.o.) und die Testverbindung werden 1 Stunde vor dem Metrazol verabreicht.

Im maximalen Elektroschock-Test für die Beurteilung der antikonvulsiven Aktivität an der Ratte, löst man einen plötzlichen Krampf-Anfall mit einem elektrischen Strom von 150 mA während 0,2 Sekunden aus. Der Strom wird durch Hornhaut-Elektroden 2 Stunden nach der Verabreichung der Testverbindung angewendet. Die Verabreichung der Testverbindung wird wie oben für den Metrazol-Test beschrieben vorgenommen. Der $ED_{50}$-Wert ist die Dosis, bei welcher die Hälfte der Tiere gegenüber dem durch Elektroschock ausgelösten Krampf-Anfall während der Beobachtungsperiode von 5 Sekunden geschützt wird.

Als Beispiele der Erfindung seien die Verbindungen der Beispiele 2, 5a und 9 genannt, welche einen $IC_{50}$-Wert von ungefähr 0,2 nM, 3 nM bzw. 0,9 nM im in vitro Benzodiazepin-Rezeptor-Test zeigen. Ferner hemmt die Verbindung des Beispiels 1 die Flunitrazepam-Bindung in vivo zu ungefähr 75% bei einer peroralen Dosis von ungefähr 30 mg/kg an der Maus.

Die Verbindungen der Erfindung wirken auch als Adenosin-Antagonisten. Eine solche Wirkung wird durch Bestimmung der Hemmung der Adenosin-Aktivierung der Adenylat-Cyclase in vesicular Präparaten aus dem Gehirn von Meerschweinchen, im wesentlichen gemäss J. Neurochem. 22, 1031 (1974), festgestellt.

Dementsprechend sind die Verbindungen der Erfindung wertvoll als auf das Nervensystem wirkende Mittel, z.B. als Benzodiazepin-Rezeptor-Modulatoren, z.B. in der Behandlung und Handhabung von Störungen des Nervensystems. Solche sind z.B. Angstzustände, konvulsive Zustände (Epilepsie) und Depression in Säugern. Die neuen Verbindungen können überdies als Zwischenprodukte zur Herstellung von anderen wertvollen, insbesondere von pharmakologisch wirksamen Präparaten, eingesetzt werden.

Die Verbindungen der Erfindung, nämlich die Verbindungen der Formel IA oder IB und ihre Salze, Derivate oder Tautomeren, werden vorzugsweise nach an sich bekannten Methoden, z.B. dadurch hergestellt, dass man

a) eine Verbindung der Formel VII

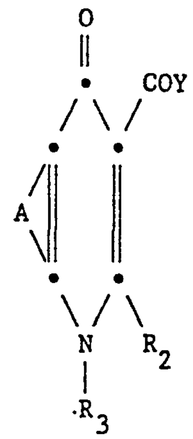

(VII),

A, $R_2$ und $R_3$ die obige Bedeutung haben und Y Niederalkoxy bedeutet mit einer Verbindung der Formel VIII $R'_3$-H-NH-$R_1$ (VIII),
worin $R_1$ die obige Bedeutung hat und $R'_3$ für Wasserstoff steht, umsetzt, oder
b) eine Verbindung der Formel IX

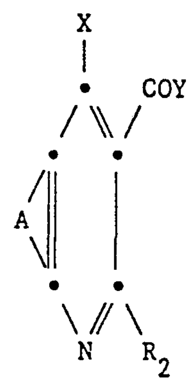

(IX),

worin A und $R_2$ die obige Bedeutung haben, X für eine reaktionsfähige veretherte oder veresterte Hydroxygruppe steht, und Y Niederalkoxy bedeutet mit einer Verbindung der Formel VIII worin $R_1$ die obige Bedeutung hat, und $R'_3$ für Wasserstoff oder Niederalkyl steht, umsetzt, oder

c) eine Verbindung der Formel IX, worin X die Gruppe -$NR'_3$-$NHR_1$- und Y Niederalkoxy oder Hydroxy bedeutet, oder X Hydroxy, ein reaktionsfähiges verestertes oder verethertes Hydroxy steht, und Y die Gruppe -$NR_1$-$NHR'_3$ bedeutet; und worin A, $R_1$, $R_2$ und $R'_3$ die obige Bedeutung haben, ringschliesst, oder

d) eine Verbindung der Formel IX, worin X Niederalkoxy, Amino oder Azido, und Y -NH-$R_1$ bedeuten, und A, $R_1$ und $R_2$ die obige Bedeutung haben, ringschliesst; oder

e) eine Verbindung der Formel X

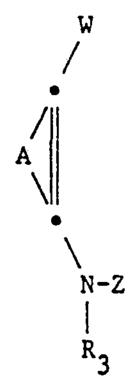

(X),

worin W und $R_3$ Wasserstoff, Z

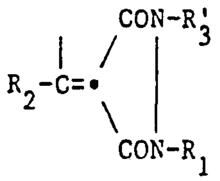

bedeuten, und A, $R_1$, $R_2$ und $R'_3$ die obige Bedeutung haben, kondensiert; oder
f) eine Verbindung der Formel X, worin W

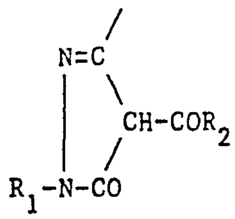

und Z Wasserstoff bedeuten und A, $R_1$, $R_2$ und $R_3$ die obige Bedeutung haben kondensiert; oder
g) eine Verbindung der Formel X, worin W

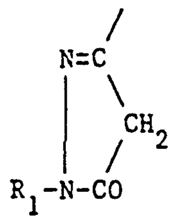

,

und Z $R_2CO$- bedeuten oder 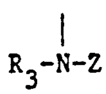

für Isocyano steht, und A, $R_1$, $R_2$ und $R_3$ die obige Bedeutung haben, kondensiert und, wenn erwünscht, eine erhaltene Verbindung der Formel IA oder IB in eine andere Verbindung der Erfindung umwandelt, und, wenn erwünscht, eine erhaltene Verbindung der Formel IA oder IB in ihr Salz oder ein erhaltenes Salz in die freie Verbindung überführt.

Die Kondensation des Verfahrens a) wird vorzugsweise bei Temperaturen zwischen ungefähr 50 und 180°, in erster Linie in Gegenwart von inerten Lösungsmitteln, z.B. aliphatischen oder aromatischen Kohlenwasserstoffen und Ethern, wie Toluol, Xylol, Biphenyl und/oder Diphenyläther, wobei das gebildete Wasser oder Alkanol abdestilliert wird, oder in Gegenwart eines Dehydratisierungsmittels, z.B. eines Molekularsiebes, durchgeführt.

Die Ausgangsstoffe der Formel VII sind bekannt oder können nach an sich bekannten Methoden, z.B. gemäss US-A- 3 429 887 und den hier beschriebenen Beispielen, hergestellt werden.

Die Ausgangsstoffe der Formel VIII sind ebenfalls bekannt oder nach an sich bekannten Methoden herstellbar.

Die Kondensation gemäss dem Verfahren b) wird mit einem Überschuss oder mit einer äquivalenten Menge einer Verbindung der Formel VIII, vorzugsweise und je nach den Reagenzien, bei einer Temperatur zwischen ungefähr und 50 und 200°, vorgenommen. Man arbeitet vorzugsweise in einem inerten Lösungsmittel, z.B. in einem Niederalkanol, wie Amylalkohol, n-Butylalkohol oder Ethanol, oder in einem aliphatischen oder aromatischen Kohlenwasserstoff, z.B. Toluol, Xylol oder Biphenyl, oder in einem aromatischen Ether, z.B. Diphenylether, oder in einem Gemisch dieser Lösungsmittel.

Die Ausgangsstoffe der Formel IX sind bekannt, oder sie werden nach an sich bekannten Methoden, z.B. gemäss US-A- 3 786 043 und den hier beschriebenen Beispielen erhalten.

In den genannten Ausgangsstoffen der Formel IX und IXa (siehe unten) steht die reaktionsfähige veresterte Hydroxygruppe X vorzugsweise für Halogen, z.B. Chlor oder Brom, Niederalkansulfonyloxy, z.B. Methansulfonyloxy. Eine reaktionsfähige veretherte Hydroxygruppe X ist vorzugsweise Niederalkoxy, z.B. Methoxy, oder Aryloxy, z.B. Phenoxy.

Der Ringschluss von Verbindungen der Formel IX, nach dem Verfahren c), wird vorzugsweise in einem

Temperaturbereich zwischen ungefähr 50 und 200°, in erster Linie in Gegenwart von inerten Lösungsmitteln, z.B. aliphatischen oder aromatischen Kohlenwasserstoffen, wie Toluol, Xylol oder Biphenyl oder von Ethern, z.B. Diphenylether, oder von Alkanolen, z.B. n-Butanol, durchgeführt. Man arbeitet je nach der Natur von X und Y, in Gegenwart oder Abwesenheit einer Base (z.B. eines Alkalimetallalkoxids, z.B. Natriumethoxid), eines Dehydratisierungsmittels (z.B. von Molekularsieben) oder eines Kondensationsmittels (z.B. n-Ethoxycarbonyl-2-ethoxy-1,2-dihydrochinolin).

Bei dem Ringschluss von Verbindungen der Formel IX, worin Y Hydroxy bedeutet, it vorteilhafterweise ein Kondensations- oder Dehydratisierungsmittel zu verwenden.

Die Ausgangsstoffe für das Verfahren c) der Formel IX, worin X für $-NR'_3-NHR_1$ steht und Y Niederalkoxy oder Hydroxy bedeutet, können durch Kondensation einer Verbindung der Formel IX, worin X eine reaktionsfähige veretherte oder veresterte Hydroxygruppe bedeutet und Y für Niederalkoxy steht, mit einem Hydrazin der Formel VIII, worin $R_1$ und $R'_3$ die vorher angegebenen Bedeutungen haben, erhalten werden. Man arbeitet in einem inerten Lösungsmittel vorzugsweise in einem Temperaturbereich zwischen ungefähr 0 und 75°, und, wenn erwünscht, hydrolysiert.

Die Hydrazid-Ausgangsstoffe der Formel IX, worin X Hydroxy, verestertes oder verethertes Hydroxy bedeutet und Y $-NR_1-NHR'_3$ ist, werden vorzugsweise durch Kondensation einer Verbindung der Formel IXa

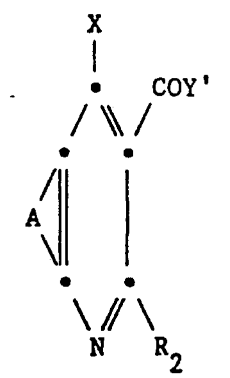

(IXa),

worin X Hydroxy, verestertes oder verethertes Hydroxy bedeutet, COY' für eine reaktionsfähige funktionell abgewandelte Carboxygruppe (z.B. Säurehalogenid oder gemischtes Anhydrid), steht und A und $R_2$ die vorher angegebene Bedeutung haben, mit einem Hydrazin der Formel VIII oder mit einem $NHR'_3$-acylierten Derivat davon (z.B. $HNR_1-NR'_3-COCF_3$), worin R lund $R'_3$ die obige Bedeutung haben, umsetzt und nachfolgend den Acylrest des erhaltenen acylierten Hydrazids abspaltet.

Ein bevorzugter Ausgangsstoff der Formel IXa ist die entsprechende ringankondensierte und substituierte Verbindung der Formel IXa, worin X und Y' Chlor bedeuten.

Der Ringschluss von Verbindungen der Formel IX, nach dem Verfahren d) wird vorzugsweise in einem Temperaturbereich zwischen ungefähr 120 und 300°, insbesondere zwischen 200 und 250°, und vorteilhafterweise auch in Gegenwart der genannten inerten Lösungsmittel durchgeführt.

Die Ausgangsstoffe für Verfahren d) der Formel IX werden vorzugsweise durch Kondensation von 4-Halogen-heterocyclo[e]pyridin-3-carbonsäurehalogeniden mit einem $R_1$-Amin und anschliessend mit einem O-Niederalkyl-hydroxylamin (ein Niederalkoxyamin) oder einem Alkalimetallazid erhalten.

Der Ringschluss von Verbindungen der Formel X nach dem Verfahren e) wird vorzugsweise mit starken aprotischen Kondensationsmitteln, wie Polyphosphorsäureniederalkylestern, vorzugsweise in Gegenwart von inerten Lösungsmitteln, wie halogenierten aliphatischen Kohlenwasserstoffen, z.B. 1,1,2,2-Tetrachlorethan durchgeführt.

Die Ausgangsstoffe für das Verfahren e) der Formel X können nach an sich bekannten Methoden hergestellt werden, z.B. durch Kondensation eines 1-Aryl-pyrazolidin-3,5-dions mit Ethylorthoformiat und einem Amino-Heterocyclus, wie 4-Aminopyridin.

Im Ringschluss von Verbindungen der Formel X gemäss dem Verfahren f) wird das gebildete Wasser vorteilhafterweise azeotropisch entfernt, vorzugsweise in Gegenwart eines konventionellen Molekularsiebes, und/oder einer katalytischen Menge einer Säure, z.B. Salzsäure.

Die Ausgangsstoffe für das Verfahren f) der Formel X können analog dem in Latvijas PSR Zinatnu Akad. Vestis. Kim. Ser. 1965 (5) 587-92 beschriebenen Verfahren hergestellt werden, angewandt auf 3-(o-Formamidoheterocyclo-pyrazol-5-one. Die genannte o-Formamidogruppe wird anschliessend mit wässeriger Säure, wie verdünnter Salzsäure, hydrolisiert.

Die ringschliessende Kondensation von Verbindungen der Formel X nach dem Verfahren g) wird vorzugsweise unter basischen Bedingungen, z.B. in Gegenwart der genannten wässerigen Alkalimetallhydroxiden oder tertiären organischen Aminen, wie Triniederalkylaminen, durchgeführt.

Die Ausgangsmaterialien für Verfahren g) der Formel X sind die $1-R_1-3$-(o-Formamidoheterocyclo)-pyrazol-5-one, die hergestellt werden können durch Kondensation von 2-(o-Formamidoheterocyclo-carbonyl)-essigsäureestern mit $R_1-NHNH_2$, und falls erwünscht, können sie mit Phosphorhalogeniden oder oxyhalogeniden zu den genannten Isonitrilen dehydratisiert werden.

Die erhaltenen Verbindungen der Erfindung können in andere Verbindungen der Formeln IA oder IB in an

sich bekannter Weise übergeführt werden. So können z.B. Verbindungen der Formel IA oder IB, worin $R_3$ oder $R'_3$ Wasserstoff bedeutet, in 1-Stellung mit einem reaktionsfähigen Ester eines Alkohols $R_3$-OH substituiert werden. Solche Ester sind z.B. $R_3$-(halogenide, sulfate, aliphatische oder aromatische sulfonate), wie Methyliodid, Dimethylsulfat, Methylmesylat oder -tosylat. Man erhält dabei 1-substituierte Verbindungen der Formel IB. Solche der Formel IA werden ähnlich, aus entsprechenden Alkalimetallsalzen, z.B. Natriumsalz, hergestellt. Man erhält dabei eine in 5-Stellung substituierte Verbindung. Die als Zwischenprodukte verwendeten Metallderivate werden durch Metallisierung mit metallorganischen Mitteln, z.B. Lithiumdiisopropylamid, mit Alkalimetallalkoxiden, z.B. Natrium-methoxid oder Thallium-(1)-ethoxid, oder mit Alkalimetallhydriden, z.B. Natrium oder Kaliumhydrid, hergestellt. Schliesslich können die Verbindungen der Erfindung entweder in freier Form oder in Form ihrer Salze erhalten werden, wann immer eine Verbindung für die Salzbildung geeignet ist. Eine erhaltene freie Base kann in das entsprechende Säureadditionssalz, vorzugsweise mit solchen Säuren oder Anionenaustauschern, welche pharmazeutisch anwendbare Salze ergeben, überführt werden. Ein erhaltenes Salz kann in die entsprechende freie Base, z.B. mit einer stärkeren Base, wie ein Metall- oder Ammoniumhydroxid oder ein basisches Salz, z.B. mit Alkalimetall-hydroxiden oder -carbonaten oder mit Kationenaustauschern, umgewandelt werden. Die Säureadditionssalze bildet man vorzugsweise mit solchen, vorher beschriebenen, anorganischen oder organischen Säuren, welche pharmazeutisch anwendbare Salze ergeben.

Verbindungen der Formel IA oder IB, worin $R_3$ oder $R'_3$ Wasserstoff bedeutet, können auch in die entsprechenden Metallsalze, z.B. durch Behandlung mit Alkalimetall- oder Erdalkalimetall-hydroxiden oder -carbonaten übergeführt werden.

Diese oder andere Salze, z.B. Pikrate, können auch in der Reinigung der erhaltenen Basen verwendet werden. Die Basen werden in ihre Salze übergeführt, die Salze abgetrennt und die freien Verbindungen aus Salzen freigesetzt.

Infolge der engen Beziehungen zwischen den neuen Verbindungen in freier Form und in Form ihrer Salze sind im vorausgegangenen und nachfolgend unter freien Verbindungen und Salzen sinn- und zweckgemäss gegebenenfalls auch die entsprechenden Salze bzw. freien Verbindungen zu verstehen.

Die Verbindungen und ihre Salze können auch in der Form ihrer Hydrate erhalten werden oder andere, zur Kristallisation verwendete Lösungsmittel, enthalten.

Erhaltene Isomerengemische der obigen Verbindungen, z.B. von solchen der Formeln I-IX können nach an sich bekannten Methoden z.B. durch fraktionierte Destillation, Kristallisation und/oder Chromatographie in die einzelnen Isomeren getrennt werden.

Die oben genannten Reaktionen werden nach an sich bekannten Methoden, in Gegenwart oder Abwesenheit von Verdünnungsmitteln, vorzugsweise in solchen, welche gegenüber den Reagenzien inert sind und diese lösen, Katalysatoren, Kondensationsmitteln oder anderen genannten Mitteln, und/oder in einer inerten Atmosphäre, unter Kühlen, bei Zimmertemperatur oder bei erhöhter Temperatur, vorzugsweise beim Siedepunkt des verwendeten Lösungsmittels, bei normalem oder erhöhtem Druck, durchgeführt.

Die Erfindung betrifft ebenfalls Abänderungen des vorliegenden Verfahrens, wonach ein auf irgendeiner Stufe des Verfahrens erhältliches Zwischenprodukt als Ausgangsmaterial verwendet wird und die verbleibenden Verfahrensschritte durchgeführt werden, oder das Verfahren auf irgendeiner Stufe abgebrochen wird, oder wonach ein Ausgangsmaterial unter den Reaktionsbedingungen gebildet, oder worin ein Ausgangsstoff in Form eines Salzes oder von reinen Isomeren verwendet wird.

Beim Verfahren der vorliegenden Erfindung werden vorzugsweise solche Ausgangsstoffe verwendet, welche zu den eingangs als besonders wertvoll geschilderten Verbindungen führen.

Die pharmakologisch verwendbaren Verbindungen der vorliegenden Erfindung können zur Herstellung von pharmazeutischen Präparaten verwendet werden, welche eine wirksame Menge der Aktivsubstanz zusammen oder im Gemisch mit Trägerstoffen enthalten, die sich zur enteralen oder parenteralen Verabreichung eignen. Vorzugsweise verwendet man Tabletten oder Gelatinekapseln, welche den Wirkstoff zusammen mit Verdünnungsmiteln, z.B. Lactose, Dextrose, Rohrzucker, Mannitol, Sorbitol, Cellulose und/oder Glycin, und Schmiermitteln, z.B. Kieselerde, Talk, Stearinsäure oder Salze davon, wie Magnesium -oder Calciumstearat, und/oder Polyethylenglykol aufweisen; Tabletten enthalten ebenfalls Bindemittel, z.B. Magnesiumalumiumsilikat, Stärke-Paste, Gelatine, Traganth, Methylcellulose, Natriumcarboxymethylcellulose und/oder Polyvinylpyrrolidon, und, wenn erwünscht, Sprengmittel, z.B. Stärkesorten, Agar, Alginsäure oder ein Salz davon, und/oder Brausemischungen, oder Absorptionsmittel, Farbstoffe, Geschmacksstoffe und Süssmittel. Injizierbare Präparate sind vorzugsweise isotonische wässerige Lösungen oder Suspensionen und Suppositorien in erster Linie Fettemulsionen oder -suspensionen. Die pharmakologischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Drucks und/oder Puffer enthalten. Die vorliegenden pharmazeutischen Präparate, die, wenn erwünscht, weitere pharmakologisch aktive Stoffe enthalten können, werden in an sich bekannter Weise, mittels konventioneller Misch-, Granulier- oder Dragierverfahren, hergestellt und enthalten etwa 0,1 bis etwa 75°%, insbesondere von 1 bis 50°%, des wirkstoffes. Einzeldosen für Säuger mit einem Gewicht von 50-70 kg können zwischen ungefähr 5 bis 100 mg des aktiven Bestandteils enthalten.

Die folgenden Beispiele dienen zur Illustration der Erfindung und sie dürfen nicht als ihre Einschränkung aufgefasst werden. Temperaturen werden in Celsiusgraden angegeben und die Angaben über Teile betreffen Gewichtsteile; wenn nicht anders definiert, wird das Eindampfen der Lösungsmittel unter vermindertem Druck,

z.B. zwischen ungefähr 15 und 100 mg Hg, durchgeführt. Für Flüssigkeiten angegebene Verhältnisse betreffen Volumenverhältnisse.

**Beispiel 1:**

Eine Lösung 2,60 g 4-Chloro-1,6-naphthyridin-3-carbon-säureethylester und 1,25 g Phenylhydrazin in 32 ml Methanol wird bei 22° 18 Stunden lang gerührt und dann während 3 Stunden am Rückfluss gekocht. Die erhaltene Aufschlämmung lässt man dann auf Raumtemperatur abkühlen, und filtriert sie anschliessend, wobei 2-Phenyl-pyrazolo[4,3-c][1,6]-naphthyridin-3-(5H)-on-hydrichlorid erhalten wird; Smp. 310°; IR (KBr) 750, 756, 777, 802, 843 $cm^{-1}$. Dieses Hydrochlorid wird in 1N Natronlauge aufgelöst und filtriert. Dem Filtrat werden 10 g Ammoniumchlorid zugefügt, der entstandene Niederschlag wird abgetrennt und aus Methanol kristallisiert, wobei die freie Base, nämlich 2-Phenyl-pyrazolo[4,3-c][1,6]naphthyridin-3(5H)-on erhalten wird; Smp. 334-337°.

Das Ausgangsmaterial wird wie folgt erhalten:

Eine Mischung von 9,3 g 4-Hydroxy-1,6-naphthyridin-3-carbonsäure-ethylester (hergestellt nach J. Org. Chem. 15, 1224 (1950) und 56 ml Phosphoroxychlorid wird während 2 Stunden 40 Minuten am Rückfluss gekocht, danach zur Trockene verdampft, und der Rückstand wird mit kaltem verdünntem Ammoniumhydroxid und Methylenchlorid behandelt. Das unlösliche Material wird durch Filtration entfernt, die organische Schicht abgetrennt, mit Salzlösung gewaschen, über Natriumsulfat getrocknet und zur Trockene eingedampft, wobei 4-Chlor-1,6-naphthyridin-3-carbonsäureethylester erhalten wird.

**Beispiel 2:**

Eine Lösung von 3,8 g 4-Chloro-1,6-naphthyridin-3-car-bonsäureethylester und 1,82 g p-Chlorphenylhydrazin in 30 ml Methanol wird bei 22° 3 Stunden gerührt und dann während 3 1/2 Stunden am Rückfluss gekocht. Die Reaktionsmischung lässt man dann auf Raumtemperatur abkühlen. Beim anschliessenden Abfiltrieren wird 2-p-Chlorphenyl-pyrazolo[4,3-c][1,6]naphthyridin-3(5H)-on-hydrochlorid erhalten; Smp. oberhalb 345°; IR (KBr) 776, 813, 823, 842, $896^{-1}$. Dieses Hydrochlorid wird wie in Beispiel 1 beschrieben in die entsprechende freie Base umgewandelt; Smp. oberhalb 360°; IR (KBr) 748, 764, 785, 790, 825, 840, 895 $cm^{-1}$.

**Beispiel 3:**

Zu einer Aufschlämmung von 1,5 g 4-Hydroxy-1,5-naphthyridin-2-carbonsäureethylester (hergestellt nach J. Inorg. Nucl. Chem., (1966) 28, 2439) in 17 ml Dimethylformamid werden 0,907 g Oxalylchlorid in 2 ml Methylenchlorid zugegeben wobei die Reaktionstemperatur zwischen -25° und -30° gehalten wird. Die Mischung wird bei -25° während 35 Minuten gerührt. Eine Lösung von 0,995 g Diisopropylethylamin und 0,833 g Phenylhydrazin in 4 ml Methylenchlorid wird der Mischung in 8 Minuten bei -30 bis -25° zugegeben. Die Mischung wird dann während 30 Minuten bei -25°, während 20 Minuten bei 0° und während 40 Minuten bei 23° gerührt. Die erhaltene dunkelrote Lösung wird in 60 ml Wasser gegossen. Der entstandene dunkle Niederschlag wird abgetrennt, mit Wasser gewaschen und aus Methanol kristallisiert, wobei 2-Phenyl-pyrazolo[4,3-c][1,5] naphthyridin-3(5H)-on erhalten wird. Smp. 314-316°.

**Beispiel 4:**

Eine Suspension von 4-[2-(4-Pyridyl)hydrazino]-1,6-naphthyridin-3-carbonsäureethylester-hydrochlorid (1,76 g) in 50 ml n-Butanol wird während 20 Stunden am Rückfluss gekocht. Die Mischung wird dann auf Raumtemperatur abgekühlt und filtriert. Der abgetrennte Feststoff wird in Natronlauge aufgelöst und bei Raumtemperatur während 21 Stunden gerührt und dann abfiltriert. Das Filtrat wird mit 1N-Salzsäure angesäuert, um einen Niederschlag auszulösen, der abgetrennt und hintereinander mit wässerigem Methanol, Methanol und Aceton gewaschen wird, wobei 2-(4-Pyridyl)-pyrazolo[4,3-c][1,6]-naphthyridin-3-(5H)-on-hydrochlorid erhalten wird; Smp. oberhalb 345°; IR (KBr) 726, 746, 791, 833, 898 $cm^{-1}$.

Das Ausgangsmaterial wird wie folgt hergestellt:

Eine Lösung von 2,36 g 4-Chloro-1,6-naphthyridin-3-carbonsäure-ethylester und 1,53 g 4-Hydrazinopyridinhydrochlorid in 37 ml Methanol wird während 19 Stunden bei Raumtemperatur gerührt und dann 4 Stunden am Rückfluss gekocht. Die Reaktionsmischung wird dann in einem Eisbad gekühlt und der Niederschlag durch Filtration abgetrennt und dann hintereinander mit Methanol und Ether gewaschen, wobei 4-[2-(4-Pyridyl)hydrazino]-1,6-naphthyridin-3-carbonsäure-ethylester-hydrochlorid erhalten wird.

**Beispiel 5:**

(a) Eine Lösung von 2,84 g 4-Chloro-1,6-naphthyridin-3-carbonsäure-ethylester und 1,35 g 3-Hydrazinopyridin in 36 ml Methanol wird bei Raumtemperatur während 2,5 Stunden gerührt, dann während 4,5 Stunden am Rückfluss gekocht, dann in einem Eisbad abgekühlt und filtriert. Der erhaltene Niederschlag wird zuerst mit Methanol und dann mit Ether gewaschen, wobei 2-(3-Pyridyl)pyrazolo[4,3-c][1,6]naphthyridin -3(5H)-on-hydrochlorid erhalten wird; Smp. 302-305°.

(b) In analoger Weise wird aus 2,6 g 4-Chlor-1,6-naphthyridin-3-carbonsäureethylester und 1,34 g 2-Hydrazinothiazol (hergestellt gemäss Can. J. Chem. 1970, 48, 3554) das 2-(2-Thiazolyl)-pyrazolo-[4,3-c][1,6]naphthyridin-3(5H)-on-hydrochlorid erhalten; Smp. oberhalb 345°; IR (KBr) 730, 746, 786, 811, 854 $cm^{-1}$.

**Beispiel 6:**

Eine Lösung von 1,75 g 4-Chlor-1,6-naphthyridin-3-carbonsäureethylester und 1,17 g 2-Hydrazinopyrimidinhydrochlorid (hergestellt gemäss Yakugakuzasshi, 1953, 73, 598) in 60 ml n-Butanol wird während 3 Stunden am Rückfluss gekocht. Die erhaltene Aufschlämmung wird auf Raumtemperatur abgekühlt und filtriert. Der abgetrennte Niederschlag wird zuerst mit Ethanol und dann mit Ether gewaschen, wobei das 2-(2-Pyrimidyl)-pyrazolo[4,3-c][1,6]naphthyridin -3(5H)-on-hydrochlorid erhalten wird; Smp. 350°; IR (KBr) 782, 791, 820, 830, 871 $cm^{-1}$.

**Beispiel 7:**

Eine Mischung von 3,0 g 4-Chlor-7-methyl-1,8-naphthyridin-3-carbonsäureethylester (hergestellt gemäss US-A-3,786,043) und 1,42 g Phenylhydrazin in 30 ml n-Butanol wird während 18 Stunden am Rückfluss gekocht. Die Mischung wird in einem Eisbad abgekühlt und filtriert. Der abgetrennte Feststoff wird in 50 ml 1N -Natronlauge und der erforderlichen Menge Wasser aufgelöst. Die erhaltene Lösung wird filtriert, um unlösliches Material abzutrennen, mit Ether gewaschen und mit 3,0 g Ammoniumchlorid neutralisiert. Die erhaltene Mischung wird filtriert, der Feststoff mit Wasser gewaschen und getrocknet, wobei 7-Methyl-2-phenyl-pyrazolo-[4,3-c][1,8]naphthyridin-3(5H)-on-hemihydrat erhalten wird, Smp. 305-308°.

**Beispiel 8:**

Eie Mischung von 2,25 g 4-Chlor-6,8-dimethoxy-1,7-naphthyridin-3-carbonsäureethylester und 1,2 g p-Chlorophenylhydrazin in 180 ml Xylol wird über Nacht am Rückfluss gekocht, dann gekühlt und mit 1N-Natronlauge (300 ml) extrahiert. Die wässerige Alkaliphase wird abgetrennt, mit Ether gewaschen und mit 20 g Ammoniumchlorid neutralisiert, wobei ein fester Niederschlag entsteht. Der Niederschlag wird abgetrennt und mit Ethanol gewaschen, wobei 2-(4-Chlor-phenyl)-6,8-dimethoxy-pyrazolo[4,3-c][1,7]naphthyridin-3(5H)-on erhalten wird, Smp. 318-321°.

Das Ausgangsmaterial wird wie folgt hergestellt:

Eine Mischung von 5,6 g 3-Amino-2,6-dimethoxypyridin und 8,8 g Diethyl-ethoxymethylenmalonat wird bei Raumtemperatur über Nacht gerührt, wobei ein Niederschlag entsteht. Der Niederschlag wird abgetrennt, in Ethylacetat aufgelöst, mit entfärbender Aktivkohle behandelt und zur Trockene eingedampft, wobei N-[3-(2,6-dimethoxypyridyl)]-aminomethylenmalonsäurediethylester entsteht; Smp. 78-81°.

N-[3-(2,6-dimethoxypyridyl)]-aminomethylenmalonsäurediethylester (12,5 g) in 250 ml einer eutektischen Mischung von Diphenylether und Biphenyl (Dowtherm®) wird während 5 Stunden unter Stickstoffatmosphäre auf Rückflusstemperatur erhitzt und dann auf Raumtemperatur abgekühlt, wobei ein Niederschlag entsteht. Der Niederschlag wird abgetrennt und mit Ether gewaschen, wobei 4-Hydroxy-6,8-dimethoxy-1,7-naphthyridin-3-carbonsäureethylester erhalten wird; Smp. 311° (Zers.).

Eine Lösung von 3,5 g Oxalylchlorid in 5 ml trockenem Acetonitril wird tropfenweise zu wasserfreiem Dimethylformamid bei -30° zugegeben. Nach 20 Minuten werden 7,0 g 4-Hydroxy-6,8-dimethoxy-1,7-naphthyridin-3-carbonsäureethylester bei -30° zugefügt. Die Reaktionstemperatur wird während 30 Minuten zwischen -20 und -30° gehalten; danach lässt man sie auf Raumtemperatur ansteigen und schliesslich wird das Gemisch zur Trockene eingedampft. Der Rückstand wird in Chloroform aufgelöst, die Lösung mit kalter wässeriger Natriumbicarbonatlösung gewaschen, über Magnesiumsulfat getrocknet, mit entfärbender Aktivkohle behandelt und zur Trockene eingedampft, wobei 4-Chlor-6,8-dimethoxy-1,7-naphthyridin-3-carbonsäureethylester erhalten wird; Smp. 200-210°.

**Beispiel 9:**

Eine Mischung von 3,2 g 4-Chlor-thieno[2,3-b]pyridin-3-carbonsäureethylester (hergestellt nach J. Heterocylic Chem., 1977, 14, 807) und 2,08 g 4-Chlorphenylhydrazin in 50 ml n-Butanol wird während 48 Stunden am Rückfluss gekocht. Die Reaktionsmischung wird in einem Eisbad abgekühlt, der entstandene Niederschlag abgetrennt und mit einer kleinen Menge n-Butanol gewaschen. Der erhaltene Feststoff wird mit Ether und 1N-Natronlauge (20 ml) und Wasser (50 ml) behandelt. Die wässerige Phase wird mit Ether gewaschen und dann mit Ammoniumchlorid (2 g) neutralisiert, wobei ein gelber Niederschlag entsteht. Der Niederschlag wird abgetrennt, mit Wasser gewaschen und getrocknet, wobei 2-(4-Chlorphenyl)-thieno-[2,3-b]pyrazolo[3,4-d]pyridin-3(5H)-on-hydrat erhalten wird; Smp. 310-313°.

**Beispiel 10:**

Die folgenden Verbindungen können analog den in den vorhergehenden Beispielen angegebenen Methoden hergestellt werden:

10/a 2-(4-Chlorphenyl)-7-methyl-pyrazolo[4,3-c][1,8]-naphthyridin-3 -(5H)on-2/3-hydrat, Smp. 340-342°.

10/b 7-Methyl-2-(3-pyridyl)-pyrazolo[4,3-c][1,8]naphthyridin-3(5H)-on-hydrat, Smp. 314-317°.

10/c 7-Methyl-2-(2-pyrimidyl)-pyrazolo[4,3-c][1,8]naphthyridin-3-(5H)-on-hydrochlorid-hemihydrat. Smp. 310-315°.

10/d 6,8-Dimethoxy-2-phenyl-pyrazolo[4,3-c][1,7]naphthyridin-3-(5H) -on, Smp. 303-306°.

**Beispiel 11:**

Verbindungen der Formeln, I, II, III, IV, V und VI, die analog den vorgenannten Beispielen hergestellt werden können:

| Beispiel | Formel | $R_1$ | andere Substituenten |
|---|---|---|---|
| 11/a | II | 4-Pyrimidyl | 7-Methyl |
| 11/b | III | 6-Me-3-pyridyl | - |
| 11/c | III | 2-Chinolyl | 8-Methyl |
| 11/d | IV | 5-MeO-2-pyrimidyl | 7-Methyl |
| 11/e | V | 5-Chlor-2-thiazolyl | 7-Chlor |
| 11/f | VI | 5-Chor-2-pyridyl | 4,6-Dimethyl |
| 11/g | VI | 5-Pyrimidyl | 4,6,7-Trimethyl |
| 11/h | VI | 4-Chlorphenyl | - |

Ausgangsmaterialien:
Beispiel

11/a 4-Hydrazinopyrimidin, J.Chem.Soc., 1955, 3478.

11/b 4-Chlor-1,7-naphthyridin-3-carbonsäureethylester aus 4-Hydroxy-1,7-naphthyridin-3-carbonsäureethylester-7-oxid, J.Org.Chem., 19, 2008 (1954)

11/c 4-chlor-6-methyl-1,7-naphthyridin-3-carbonsäureethylester aus 4-Hydroxy-6-methyl-1,7-naphthyridin-3-carbonsäureethylester, US-A- 3,429,887.

11/d 5-Methoxy-2-hydrazinopyrimidin von 2-Chlor-5-methoxy-pyri-midin, J.Chem.Soc., 1960, 4590.

4-chlor-2-methyl-thieno[2,3-b]pyridin-5-carbonsäureethylester aus 4-Hydroxy-2-methyl-thieno[2,3-b]pyridin-5-carbonsäureethylester, US-A- 3,997,545.

11/e 2,7-Dichlor-thieno[3,2-b]pyridin-6-carbonsäureethylester, EP-A- 46,990.

11/f 7-Chlor-3,5-dimethyl-imidazo[4,5-b]pyridin-6-carbonsäureethylester. J.Heterocyclic Chem., 14, 813 (1977).

11/g 7-chlor-2,3,5-trimethyl-imidazo[4,5-b]pyridin-6-carbonsäureethylester, J.Heterocyclic Chem., 14, 813 (1977).

**Beispiel 12:**

Durch die Zugabe von Lithiumhydroxid wird das pH der Lösung von 0,5 g 4-Chlor-[1,6]naphthyridin-3-(n-phenyl-N-trifluoracetamido)carboxamid in der minimalen Menge von 50%igem wässerigem Tetrahydrofuran auf 11 eingestellt. Die Mischung wird bei Raumtemperatur während 48 Stunden gerührt, eingedampft, um den Grossteil des Tetrahydrofurans zu entfernen, mit Diethylether gewaschen und durch Zugabe von verdünnter Salzsäure auf ein pH von 6 neutralisiert. Der gebildete Niederschlag wird durch Saugfiltration abgetrennt und durch präparative Dünnschichtchromatographie gereinigt unter Verwendung von Toluol-Ethanol-

konz.Ammoniak (70:30:3) als entwickelndes Lösungsmittel auf Silicagel, um 2-Phenylpyrazolo[4,3-c]-[1,6]naphthyridin-3(5H)-on zu erhalten; Smp. 334-337°.

Das Ausgangsmaterial wird wie folgt hergestellt: 4-Hydroxy[1,6]-naphthyridin-3-carbonsäureethylester wird in 4-Chlor-3-chlorcarbonyl [1,6]-naphthyridinhydrochlorid umgewandelt durch Hydrolyse des Esters zur entsprechenden 3-Carbonsäure und anschliessender Behandlung der genannten Säure mit Phosphoroxychlorid. Die Mischung von 1,5 g β-Trifluoracetyl-phenylhydrazin [N. Yokoyama, U.S. Patent 4 312 870 (1982)] und 0,06 g Lithiumhydrid in 100 ml Tetrahydrofuran wird unter Feuchtigkeitsausschluss während 5 Stunden bei Raumtemperatur gerührt, bis eine Lösung entsteht. Getrennt davon werden 1,9 g 4-Chlor-4-chlorcarbonyl[1,6]naphthyridinhydrochlorid in 100 ml Tetrahydrofuran mit 0,06 g Lithiumhydrid unter Feuchtigkeitsausschluss während 1 Minute bei 10° gerührt und die Lösung der eben beschriebenen zugefügt und zwar in Portionen von 10 ml. Die Mischung wird bei Raumtemperatur während 24 Stunden gerührt, danach während 8 Stunden am Rückfluss gekocht und unter reduziertem Druck eingedampft, wobei das 4-Chlor-[1,6]naphthyridin-3-(N-phenyl-N-trifluor-acetamido)carboxamid erhalten wird, welches ohne weitere Reinigung weiterverwendet wird.

**Beispiel 13**:

Die Mischung von 0,3 g 4-(O-Methylhydroxylamino)-[1,6]naphthyridin-3-(N-phenyl)carboxamid und 15 ml eutektisches Diphenylether-Biphenyl wird während 2 Stunden unter Stickstoff auf 240° erhitzt. Sie wird dann auf Raumtemperatur abgekühlt und unter Hochvakuum eingedampft. Der Rückstand wird mit 100 ml Petrolether verdünnt und der entstehende Niederschlag abgetrennt. Er wird mit Petroleumether gewaschen, mit 15 ml Diethylether und 3 ml 2N wässeriger Natronlauge während 1 Stunde gerührt, filtriert, um das unlösliche Material abzutrennen, und die Filtratschichten werden getrennt. Die wässerige Phase wird mit 0,32 g Ammoniumchlorid behandelt, wobei ein gelber Niederschlag erhalten wird, der abgetrennt und aus Methanol umkristallisiert wird, um 2-Phenylpyrazolo-[4,3-c][1,6]naphthyridin-3(5H)-on zu erhalten; Smp. 335-337°.

Das Ausgangsmaterial wird wie folgt erhalten: Die Mischung von 9,5 g 4-Hydroxy[1,6]naphthyridin-3-carbonsäure, 4,7 g Anilin, 12,4 g 1-Ethoxycarbonylformamid wird während 24 Stunden auf 60-70° erhitzt, um eine klare Lösung zu erhalten. Diese wird abgekühlt und unter reduziertem Druck eingedampft. Der Rückstand wird mit Diethylether trituriert, filtriert und der abgetrennte Niederschlag wird nacheinander mit Diethylether, kalter 2N wässeriger Natronlauge und dann mit Wasser gewaschen und getrocknet, um das 4-Hydroxy-[1,6]naphthyridin-3-(N-phenyl)-carboxamid zu erhalten. Die Mischung von 1,0 g davon und 25 ml Phpophoroxychlorid wird während 3 Stunden auf 80° erhitzt, um eine klare Lösung zu erhalten. Diese wird eingedampft, der Rückstand mit 400 ml einer 1:1-Mischung von eiskalter 2N wässeriger Natronlauge und Dichlormethan behandelt. Die organische Phase wird abgetrennt, getrocknet und eingedampft, wobei das 4-Chlor-[1,6]naphthyridin-3-(N-phenyl)-carboxamid erhalten wird. Die Mischung von 0,5 g davon, 1,0 g O-Methylhydroxylaminhydrochlorid und 1,65 g Diisopropylethylamin wird in einem kleinen Druckgefäss während 18 Stunden auf 100° erhitzt. Die gekühlte Mischung wird dann mit Wasser trituriert, in Tetrahydrofuran aufgelöst, getrocknet und eingedampft, wobei das 4-(O-Methylhydroxylamino)-[1,6]naphthyridin-3-(N-phenyl)carboxamid erhalten wird.

**Beispiel 14**:

Die Mischung von 0,5 g 4-(4-Pyridylaminomethyliden)-1-phenylpyrazolidin-3,5-dion, 2,0 g Ethylpolyphosphat und 10 ml 1,1,2,2-Tetrachlorethan wird während 24 Stunden am Rückfluss gekocht. Die Lösung wird unter Rühren auf 30 ml Eiswasser gegossen. Danach wird das pH der wässerigen Phase durch Zufügen von 2N wässeriger Natronlauge auf 6 eingestellt, und mit Ethylacetat (30 ml Portionen) extrahiert. Die organische Phase wird mit 15 ml 2N wässeriger Natronlauge extrahiert. Die alkalische wässerige Phase wird mit 1,6 g Ammoniumchlorid neutralisiert, wobei ein gelber Niederschlag entsteht, der abgetrennt und aus Isopropanol umkristallisiert wird, um das 2-Phenylpyrazolo[4,3-c][1,6]napthyridin-3(5H)-on zu erhalten; Smp. 334-336°.

Das Ausgangsmaterial wird wie folgt hergestellt: Zu 20,0 g Diethylmalonat werden 5,8 g Natriummetall zugegeben, das in 100 ml abs. Ethanol gelöst ist. Nach 15-minütigem Rühren werden 13,5 g Phenylhydrazin zugefügt und die entstehende Mischung wird vom überflüssigen Ethanol befreit. Der Rückstand wird während 5 Stunden auf 110-120° erhitzt und dann mit 500 ml Eiswasser abgeschreckt. Die resultierende Mischung wird mit Diethylether gewaschen und die wässerige Schicht wird mit konz. Salzsäure-angesäuert auf ein pH unter 2. Der entstehende Niederschlag wird aus Toluol umkristallisiert, um das 1-Phenylpyrazolidin-3,5-dion zu erhalten.

Die Mischung von 2,0 g davon, 2,8 g Triethylorthoformat, 1,0 g 4-Aminopyridin und 30 ml Ethanol wird während 16 Stunden am Rückfluss gekocht. Sie wird dann gekühlt, filtriert und der abgetrennte Niederschlag wird mit Ethanol gewaschen, um das 4-(4-Pyridylaminomethyliden)-1-phenylpyrazolidin-3,5-dion zu erhalten.

**Beispiel 15:**

Die Lösung von 50 mg 3-(4-Formamido-3-pyridyl)-1-phenyl-4,5-dihydropyrazol-5-on in 10 ml Dichlormethan wird eingedampft, wobei ein dünner Film an der Flaschenwand zurückbleibt. Dieser wird unter einem sanften Stickstoffstrom auf 190-200° während 30 Minuten erhitzt. Das Reaktionsprodukt wird durch praparative Dünnschichtchromatographie gereinigt unter Verwendung von Toluol:-Ethanol:konz.Ammoniak (70:30:3) als entwickelndes Lösungsmittel auf Silicagel, um das 2-Phenylpyrazolo-[4,3-c][1,6]naphthyridin-3(5H)-on zu erhalten; Smp. 334-336°.

Das Ausgangsmaterial wird wie folgt hergestellt: 1,5 g Methyl-4-amino-nicotinat [T. Itai et al., Yakugakuzashi 75, 292 (1955)] werden zu 20 ml 97%iger Ameisensäure unter Eiskühlung und Rühren unter Stickstoffatmosphäre zugefügt, um eine klare Lösung zu erhalten. Danach werden 2 ml Essigsäureanhydrid zugefügt, die Mischung über Nacht bei Raumtemperatur gerührt und dann in 300 ml Sole gegossen. Der Niederschlag wird mit Dichlormethan extrahiert. Die organische Phase wird getrocknet und zur Trockene eingedampft, wobei der 4-Formamido-nicotinsäuremethylester zurückbleibt.

Danach werden 2,07 g Methylacetat in 2 ml Tetrahydrofuran einer Suspension von 1,34 g Natriumhydroxid (50%ige Dispersion in Mineralöl) in 25 ml Tetrahydrofuran bei 0-5° zugegeben. Wenn die Zugabe vollständig ist, wird die Mischung am Rückfluss während einer Stunde gekocht, dann in einem Eisbad gekühlt und 4,7 g des oben genannten 4-Formamido-nicotinsäuremethylesters in 5 ml Tetrahydrofuran bei 5° zugegeben. Nach der Zugabe wird die Mischung während 2 Stunden am Rückfluss gekocht, auf Raumtemperatur abgekühlt und in Wasser gegossen. Die wässerige Phase wird abgetrennt, mit Diethylether gewaschen, durch Zugabe von 2,0 g Ammoniumchlorid neutralisiert und mit Diethylether extrahiert. Der etherische Extrakt wird getrocknet und eingedampft, wobei der rohe 2-(4-Formamido-3-pyridylcarbonyl)-essigsäuremethylester erhalten wird. Die Lösung von 2,2 g davon und 1,2 g Phenylhydrazin in 75 ml Toluol wird während 6 Stunden am Rückfluss mit einem Wasserabscheider gekocht. Die Mischung wird eingedampft, der Rückstand chromatographiert auf Silicagel mit 5%-igem Ethylacetat in Toluol als Eluierungsmittel, wobei das 3-(4-Formamido-3-pyridyl)-1-phenyl-4,5-dihydropyrazol-5-on erhalten wird.

**Beispiel 16:**

Einer Lösung von 0,5 g 3-(4-Amino-3-pyridyl)-4-hydroxy-methylen-1-phenyl-4,5-dihydropyrazol-5-on in 150 ml Toluol werden 0,1 ml konz. Salzsäure zugefügt und die Mischung wird mit einem Wasserabscheider während 24 Stunden am Rückfluss gekocht. Sie wird gekühlt, der entstandene Niederschlag abgetrennt und durch präparative Dünnschichtchromatographie gereinigt unter Verwendung von Toluol:Ethanol:konz.Ammoniak (70:30:3) als entwickelndes Lösungsmittel auf Silicagel, wobei 2-Phenyl-pyrazolo[4,3-c][1,6]naphthyridin-din-3(5H)-on erhalten wird; Smp. 334-335°.

Das Ausgangsmaterial wird wie folgt hergestellt: 3,0 g des genannten 3-(4-Formamido-3-pyridyl)-1-phenyl-4,5-dihydropyrazol-5-ons wird während 24 Stunden bei Raumtemperatur in 20 ml Dimethylformamiddimethylacetal gerührt. Die dunkle Reaktionsmischung wird auf Eiswasser gegossen. Der Niederschlag wird durch Saugfiltration abgetrennt, in Ethylacetat aufgenommen, mit Wasser gewaschen, getrocknet und zur Trockene eingedampft. wobei das 4-Dimethylamino-methylen-3-(4-formamido-3-pyridyl)-1-phenyl-4,5-dihydropyrazol-5-on erhalten wird. Eine Mischung von 2,5 g davon in 20 ml Tetrahydrofuran und 20 ml 20%iger wässeriger Salzsäure wird während 4 Stunden bei 60° und dann während 16 Stunden bei Raumtemperatur gerührt. Diese Mischung wird mit Sole verdünnt, das pH durch Zugabe von wässeriger Natronlauge auf 6 eingestellt und mit Dichlormethan extrahiert. Die organische Phase wird abgetrennt, getrocknet und zur Trockene eingedampft, wobei das 3-(4-Amino-3-pyridyl)-4-hydroxymethyle-1-phenyl-4,5-dihydropyrazol-5-on erhalten wird.

**Beispiel 17:**

Aus den folgenden Bestandteilen werden 10'000 Tabletten hergestellt, wobei jede Tablette 10 mg Wirkstoff enthält:

| | |
|---|---|
| 2-(3-Pyridyl)-pyrazolo[4,3-d][1,6]-naphthyridin-3(5H)-on | 100,00 g |
| Lactose | 1,157,00 g |
| Maisstärke | 75,00 g |
| Polyethylenglykol 6000 | 75,00 g |
| Talkpulver | 75,00 g |
| Magnesiumstearat | 18,00 g |
| Wasser | q.s. |

Verfahren:

Die Mischung der oben genannten Bestandteile wird durch ein Sieb mit der Maschenweite von 0,6 mm getrieben. Danach wird der Wirkstoff, die Lactose, das Talkpulver, das Magnesiumstearat und die Hälfte der

Stärkemenge mechanisch gut vermischt. Die andere Hälfte der Stärkemenge wird in 40 ml Wasser suspendiert und die Suspension der kochenden Lösung des Polyethylenglykols in 150 ml Wasser zugefügt. Die entstehende Paste wird der pulverförmigen Mischung zugegeben und das Ganze dann granuliert, wenn nötig unter weiterem Wasserzusatz. Das Granulat wird über Nacht bei 35° getrocknet, gemahlen über einem Sieb mit 1,2 mm Maschenöffnung und dann zu Tabletten verpresst, wobei konkave Stempel mit 6,4 mm Durchmesser (oberer Stempel mit Teilkerbe) verwendet werden.

**Beispiel 18:**

Aus den folgenden Bestandteilen werden 10'000 Kapseln hergestellt, wobei jede Kapsel 25 mg Wirksubstanz enthält:

| | |
|---|---|
| 2-(4-Chlorphenyl)-thieno[2,3-b]-pyrazolo[3,4-d]pyridin-3(5H)-on | 250,0 g |
| Lactose | 1,650,0 g |
| Talkpulver | 100,0 g |

Verfahren:
Die pulverförmigen Substanzen werden durch ein Sieb mit einer Maschenöffnung von 0,6 mm getrieben. Danach wird der Wirkstoff in einen Mixer gegeben und zuerst mit dem Talkpuder und dann mit der Lactose gründlich vermischt. Unter Verwendung einer Kapselfüllmaschine wird jede Kapsel (Nr. 3) mit 200 g der erhaltenen Mischung gefüllt.

Analoge Tabletten oder Kapseln können mit den übrigen erfindungsgemässen Verbindungen hergestellt werden, insbesondere mit denjenigen gemäss den Beispielen.

**Patentansprüche**

für die Vertragsstaaten: BE, CH, DE, FR, GB, IT, LI, LU, NL, SE

1. 2-Substituierte-[b]-heterocyclisch ankondensierte Pyrazolo-[3,4-d]-pyridin-3-one der Formeln IA oder IB

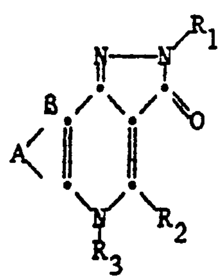
(IA) oder
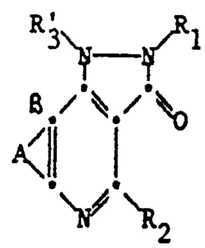
(IB)

worin A zusammen mit den 2 Kohlenstoffatomen, an die es gebunden ist, einen ankondensierten ungesättigten heterocyclischen Ring darstellt aus der Gruppe (a) Thieno, Furo und gegebenenfalls N-Niederalkyl substituiertes Pyrrolo, wobei die Kohlenstoffatome in jedem dieser Ringe unsubstituiert oder eines von ihnen durch Niederalkyl, Carbo-(nieder)-alkoxy, Halogen oder Trifluormethyl substituiert ist; (b) gegebenenfalls N-Niederalkyl substituiertes Imidazo, worin das Kohlenstoffatom im genannten Ring gegebenenfalls durch Niederalkyl, Niederalkoxy, Halogen oder Trifluormethyl substituiert ist; (c) Thiazolo, Oxazolo, Isoxazolo, 2,3-, 3,4- oder 4,3-Pyrido und Pyridazino, wobei die Kohlenstoffatome, die diese Ringe bilden, unsubstituiert sind oder eines oder zwei davon durch Niederalkyl, Niederalkoxy oder Halogen substituiert sind; (d) Pyrimido und Pyrazino, wobei die Kohlenstoffatome in diesen Ringen unsubstituiert oder eines davon durch Niederalkyl oder Niederalkoxy substituiert ist; $R_1$ gegebenenfalls durch Niederalkyl, Niederalkoxy, Halogen oder Trifluoromethyl mono- oder disubstituiertes Phenyl bedeutet; oder $R_1$ einen aromatischen heterocyclischen Rest aus der Gruppe Pyridyl, Chinolyl, Isochinolyl, Pyrimidyl und Thiazolyl oder jeden der genannten heterocyclischen Reste bedeutet, die durch Niederalkyl, Niederalkoxy oder Halogen mono- oder disubstituiert sind; und $R_2$, $R_3$ und $R'_3$ unabhängig voneinander Wasserstoff oder Niederalkyl bedeuten; wobei die mit "nieder" bezeichneten Reste höchstens 7 Kohlenstoffatome enthalten, ihre Tautomeren, oder Salze davon.

2. Verbindungen der Formeln (IA) oder (IB), gemäss Anspruch 1, worin A zusammen mit den beiden Kohlenstoffatomen, an welche es gebunden ist, ankondensierte 2,3-, 3,4- oder 4,3-Pyrido oder jeden der genannten Pyridoringe darstellt, die durch Niederalkyl, Niederalkoxy oder Halogen mono- oder disubstituiert sind; wobei die mit "nieder" bezeichneten Reste höchstens 7 Kohlenstoffatome enthalten, und $R_1$, $R_2$, $R_3$ und $R'_3$ die in Anspruch 1 angegebenen Bedeutungen haben; oder Tautomere davon oder pharmazeutisch verwendbare Salze davon.

3. Verbindungen der Formel II gemäss Anspruch 1

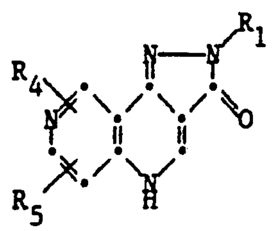

(II),

worin $R_1$ gegebenenfalls durch Niederalkyl, Niederalkoxy, Halogen oder Trifluoromethyl mono- oder disubstituiertes Phenyl darstellt; oder $R_1$ einen aromatischen heterocyclischen Rest aus der Gruppe Pyridyl, Chinolyl, Isochinolyl, Pyrimidyl oder Thiazolyl darstellt, oder einen dieser genannten Reste, der durch Niederalkyl, Niederalkoxy oder Halogen mono- oder disubstituiert ist; $R_4$ und $R_5$ unabhängig voneinander Wasserstoff, Niederalkyl, Niederalkoxy oder Halogen darstellen; wobei die mit "nieder" bezeichneten Reste höchstens 7 Kohlenstoffatome enthalten, oder Tautomere davon, oder pharmazeutisch annehmbare Salze davon.

4. Verbindungen der Formel III gemäss Anspruch 1

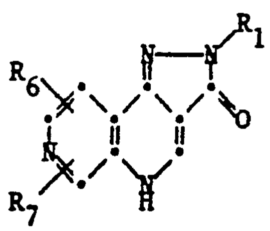

(III),

worin $R_1$ gegebenenfalls durch Niederalkyl, Niederalkoxy, Halogen oder Trifluoromethyl mono- oder disubstituiertes Phenyl bedeutet; oder $R_1$ einen aromatischen heterocyclischen Rest aus der Gruppe Pyridyl, Chinolyl, Isochinolyl, Pyrimidyl und Thiazolyl bedeutet, oder einen der genannten Reste, der durch Niederalkyl, Niederalkoxy oder Halogen mono- oder di-substituiert ist; $R_6$ und $R_7$ unabhängig voneinander Wasserstoff, Niederalkyl, Niederalkoxy oder Halogen bedeuten; wobei die mit "nieder" bezeichneten Reste höchstens 7 Kohlenstoffatome enthalten, oder Tautomere davon; oder pharmazeutisch annehmbare Salze davon.

5. Verbindungen der Formeln IA oder IB, gemäss Anspruch 1, worin A zusammen mit den zwei Kohlenstoffatomen, an welche es gebunden ist, ankondensiertes 2,3- oder 3,2-Thieno darstellt, und wobei die Kohlenstoffatome im genannten Thienoring unsubstituiert sind oder einer davon durch Niederalkyl, Carbo(nieder)-alkoxy, Halogen oder Trifluoromethyl substituiert ist, wobei die mit "nieder" bezeichneten Reste höchstens 7 Kohlenstoffatome enthalten, und $R_1$, $R_2$, $R_3$ und $R'_3$ die im Anspruch 1 angegebene Bedeutung haben; oder pharmazeutisch annehmbare Salze davon.

6. Verbindungen der Formeln (IA) oder (IB), gemäss Anspruch 1, worin A zusammen mit den zwei Kohlenstoffatomen, an welche es gebunden ist, gegebenenfalls N-Niederalkyl substituiertes ankondensiertes 5,4-Imidazo darstellt, und das Kohlenstoff im genannten 5,4-Imidazoring gegebenenfalls durch Niederalkyl, oder gegebenenfalls durch Niederalkyl, Niederalkoxy, Halogen oder Trifluoromethyl mono-substituiertes Phenyl substituiert ist, wobei die mit "nieder" bezeichneten Reste höchstens 7 Kohlenstoffatome enthalten, und $R_1$, $R_2$, $R_3$ und $R'_3$ die im Anspruch 1 angegebene Bedeutung haben; oder pharmazeutisch verwendbare Salze davon.

7. 2-Phenyl-pyrazolo-[4,3-c][1,6]naphthyridin-3(5H)-on oder pharmazeutisch verwendbare Salze davon.

8. 2-(3-Pyridyl)-pyrazolo[4,3-c][1,6]naphthyridin-3(5H)-on oder pharmazeutisch verwendbare Salze davon.

9. 2-(2-Thiazolyl)-pyrazolo[4,3-c][1,6]naphthtyridin-3(5H)-on oder pharmazeutisch verwendbare Salze davon.

10. 2-p-Chlorphenyl-pyrazolo[4,3-c][1,7]naphthyridin-3(5H)-on oder pharmazeutisch verwendbare Salze davon.

11. Pharmazeutische Präparate enthaltend eine Verbindung der Ansprüche 1-10, zusammen mit einem pharmazeutischen Trägermaterial.

12. Die in den Ansprüchen 1-10 genannten Verbindungen zur Anwendung in einem Verfahren zur therapeutischen Behandlung des menschlichen oder tierischen Körpers.

13. Verwendung der in den Ansprüchen 1-10 genannten Verbindungen zur Herstellung eines Arzneimittels mit Benzodiazepin-Rezeptoren modulierenden Eigenschaften.

14. Verwendung der in den Ansprüchen 1-10 genannten Verbindungen zur Herstellung von pharmazeutischen Präparaten.

15. Verfahren zur Herstellung der im Anspruch 1 genannten Verbindungen der Formeln (IA) oder (IB), dadurch gekennzeichnet, dass man

a) eine Verbindung der Formel VII

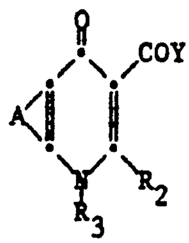

(VII),

worin A, $R_2$ und $R_3$ die im Anspruch 1 angegebene Bedeutung haben und Y Niederalkoxy bedeutet, mit einer Verbindung der Formel VIII

$R'_3$-NH-NH-$R_1$ (VIII),

worin $R_1$ die in Anspruch 1 angegebene Bedeutung hat und $R'_3$ für Wasserstoff steht, umsetzt, oder

b) eine Verbindung der Formel IX

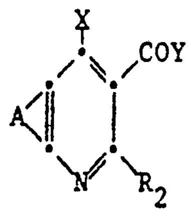

(IX),

worin A und $R_2$ die im Anspruch 1 angegebene Bedeutung haben, X für eine reaktionsfähige veretherte oder veresterte Hydroxygruppe steht, und Y Niederalkoxy bedeutet, mit einer Verbindung der Formel VIII, worin $R_1$ die in Anspruch 1 angegebene Bedeutung hat, und $R'_3$ für Wasserstoff oder Niederalkyl steht, umsetzt, oder

c) eine Verbindung der Formel IX, worin X die Gruppe -$NR'_3$-$NHR_1$,Y Niederalkoxy oder Hydroxy bedeutet, oder X Hydroxy, ein reaktionsfähiges verestertes oder verethertes Hydroxy steht, und Y die Gruppe -$NR_1$-$NHR'_3$ bedeutet; und worin A, $R_1$, $R_2$ und $R'_3$ die im Anspruch 1 angegebene Bedeutung haben, ringschliesst; oder

d) eine Verbindung der Formel IX, worin X Niederalkoxy, Amino oder Azido, und Y -NH-$R_1$ bedeuten, und A, $R_1$ und $R_2$ die im Anspruch 1 angegebene Bedeutung haben, ringschliesst; oder

e) eine Verbindung der Formel X

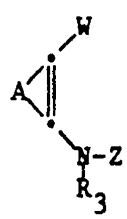

(X),

worin W und $R_3$ Wasserstoff, Z

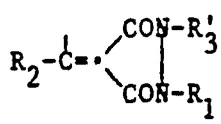

bedeuten, und A, $R_1$, $R_2$ und $R'_3$ die im Anspruch 1 angegebene Bedeutung haben, kondensiert; oder

f) eine Verbindung der Formel X, worin W

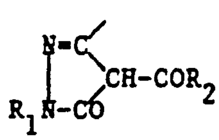

und Z Wasserstoff bedeuten, und A, $R_1$, $R_2$ und $R_3$ die im Anspruch 1 angegebene Bedeutung haben, kondensiert; oder

g) eine Verbindung der Formel X, worin W

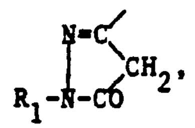

und Z $R_2CO$- bedeuten oder $R_3-N-Z$

für Isocyano steht, und A, $R_1$, $R_2$ und $R_3$ die im Anspruch 1 angegebene Bedeutung haben, kondensiert, wenn erwünscht, in allen diesen Verfahren eine störende reaktionsfähige Gruppe vorübergehend schützt, und die erhaltene Verbindung der Formel IA oder IB isoliert; und, wenn erwünscht, eine erhaltene Verbindung der Formel IA oder IB in eine sndere erfindungsgemässe Verbindung umwandelt, und/oder, wenn erwünscht, eine erhaltene Verbindung in ihr Salz oder·ein erhaltenes Salz in die freie Verbindung oder in ein anderes Salz überführt, und/oder, wenn erwünscht, ein erhaltenes Gemisch von Isomeren oder Razematen in die einzelnen Isomeren oder Razemate auftrennt, und/oder, wenn erwünscht, erhaltene Razemate in die optischen Antipoden trennt.

16. Die nach dem Verfahren des Anspruches 15 erhältlichen Verbindungen.

**Patentansprüche**

für den Vertragsstaat AT

1. Verfahren zur Herstellung von neuen 2-substituierten-[b]-heterocyclisch ankondensierten Pyrazolo[3,4-d]-pyridin-3-onen der Formeln IA oder IB

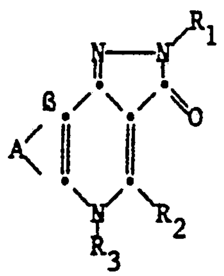 (IA) oder 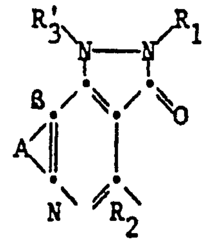 (IB)

worin A zusammen mit den 2 Kohlenstoffatomen, an die es gebunden ist, einen ankondensierten ungesättigten heterocyclischen Ring darstellt aus der Gruppe (a) Thieno, Furo und gegebenenfalls N-Niederalkyl substituiertes Pyrrolo, wobei die Kohlenstoffatome in jedem dieser Ringe unsubstituiert oder eines von ihnen durch Niederalkyl, Carbo-(nieder)-alkoxy, Halogen oder Trifluormethyl substituiert ist; (b) gegebenenfalls N-Niederalkyl substituiertes Imidazo, worin das Kohlenstoffatom im genannten Ring gegebenenfalls durch Niederalkyl, Niederalkoxy, Halogen oder Trifluormethyl substituiert ist; (c) Thiazolo, Oxazolo, Isoxazolo, 2,3-, 3,4- oder 4,3-Pyrido und Pyridazino, wobei die Kohlenstoffatome, die diese Ringe bilden, unsubstituiert sind oder eines oder zwei davon durch Niederalkyl, Niederalkoxy oder Halogen substituiert sind; (d) Pyrimido und Pyrazino, wobei die Kohlenstoffatome in diesen Ringen unsubstituiert oder eines davon durch Niederalkyl oder Niederalkoxy substituiert ist; $R_1$ gegebenenfalls durch Niederalkyl, Niederalkoxy, Halogen oder Trifluoromethyl mono- oder disubstituiertes Phenyl bedeutet; oder $R_1$ einen aromatischen heterocyclischen Rest aus der Gruppe Pyridyl, Chinolyl, Isochinolyl, Pyrimidyl und Thiazolyl oder jeden der genannten heterocyclischen Reste bedeutet, die durch Niederalkyl, Niederalkoxy oder Halogen mono- oder disubstituiert sind; und $R_2$, $R_3$ und $R'_3$ unabhängig voneinander Wasserstoff oder Niederalkyl bedeuten, wobei die mit "nieder" bezeichneten Reste höchstens 7 Kohlenstoffatome enthalten, oder Tautomere davon, oder Salze davon, dadurch gekennzeichnet, dass man

a) eine Verbindung der Formel VII

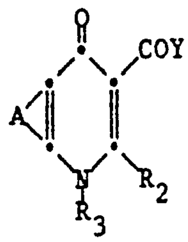

(VII),

worin A, $R_2$ und $R_3$ die oben angegebene Bedeutung haben und Y Niederalkoxy bedeutet, mit einer Verbindung der Formel VIII

$R'_3$-NH-NH-$R_1$ (VIII),

worin $R_1$ die oben angegebene Bedeutung hat und $R'_3$ für Wasserstoff steht, umsetzt, oder

b) eine Verbindung der Formel IX

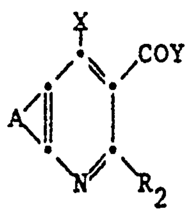

(IX),

worin A und $R_2$ die oben angegebene Bedeutung haben, X für eine reaktionsfähige veretherte oder veresterte Hydroxygruppe steht, und Y Niederalkoxy bedeutet, mit einer Verbindung der Formel VIII, worin $R_1$ die oben angegebene Bedeutung hat, und $R'_3$ für Wasserstoff oder Niederalkyl steht, umsetzt, oder

c) eine Verbindung der Formel IX, worin X die Gruppe -$NR'_3$-$NHR_1$,Y Niederalkoxy oder Hydroxy bedeutet, oder X Hydroxy, ein reaktionsfähiges verestertes oder verethertes Hydroxy steht, und Y die Gruppe -$NR_1$-$NHR'_3$ bedeutet; und worin A, $R_1$, $R_2$ und $R'_3$ die oben angegebene Bedeutung haben, ringschliesst; oder

d) eine Verbindung der Formel IX, worin X Niederalkoxy, Amino oder Azido, und Y -NH-$R_1$ bedeuten, und A, $R_1$ und $R_2$ die oben angegebene Bedeutung haben, ringschliesst; oder

e) eine Verbindung der Formel X

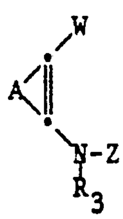

(X),

worin W und $R_3$ Wasserstoff, Z

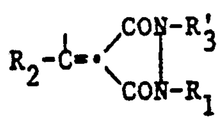

bedeuten, und A, $R_1$, $R_2$ und $R'_3$ die oben angegebene Bedeutung haben, kondensiert; oder

f) eine Verbindung der Formel X, worin W

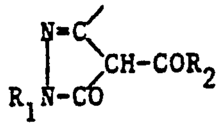

und Z Wasserstoff bedeuten, und A, $R_1$, $R_2$ und $R_3$ die oben angegebene Bedeutung haben, kondensiert; oder

g) eine Verbindung der Formel X, worin W

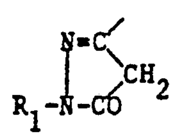 ,

und Z $R_2CO$- bedeuten oder $R_3-\overset{|}{N}-Z$

für Isocyano steht, und A, $R_1$, $R_2$ und $R_3$ die oben angegebene Bedeutung haben, kondensiert, wenn erwünscht, in allen diesen Verfahren eine störende reaktionsfähige Gruppe vorübergehend schützt, und die erhaltene Verbindung der Formel IA oder IB isoliert; und, wenn erwünscht, eine erhaltene Verbindung der Formel IA oder IB in eine andere erfindungsgemässe Verbindung umwandelt, und/oder, wenn erwünscht, eine erhaltene Verbindung in ihr Salz oder ein erhaltenes Salz in die freie Verbindung oder in ein anderes Salz überführt, und/oder, wenn erwünscht, ein erhaltenes Gemisch von Isomeren oder Razematen in die einzelnen Isomeren oder Razemate auftrennt, und/oder, wenn erwünscht, erhaltene Razemate in die optischen Antipoden trennt.

2. Verfahren nach Anspruch 1, dadurch gekennzeichnet, dass man Verbindungen der im Anspruch 1 gezeigten Formeln IA und IB, worin A zusammen mit den beiden Kohlenstoffatomen, an welche es gebunden ist, ankondensierte 2,3-, 3,4- oder 4,3-Pyrido oder jeden der genannten Pyridoringe darstellt, die durch Niederalkyl, Niederalkoxy oder Halogen mono- oder disubstituiert sind; wobei die mit "nieder" bezeichneten Reste höchstens 7 Kohlenstoffatome enthalten, und $R_1$, $R_2$, $R_3$ und $R'_3$ die in Anspruch 1 angegebenen Bedeutungen haben; oder Salze davon herstellt.

3. Verfahren nach Anspruch 1, dadurch gekennzeichnet, dass man Verbindungen der Formel II

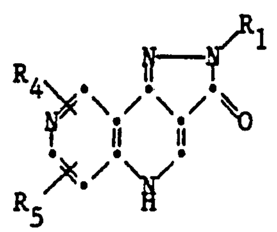 (II),

worin $R_1$ gegebenenfalls durch Niederalkyl, Niederalkoxy, Halogen oder Trifluoromethyl mono- oder disubstituiertes Phenyl darstellt; oder $R_1$ einen aromatischen heterocyclischen Rest aus der Gruppe Pyridyl, Chinolyl, Isochinolyl, Pyrimidyl oder Thiazolyl darstellt, oder einen dieser genannten Reste, der durch Niederalkyl, Niederalkoxy oder Halogen mono- oder disubstituiert ist; $R_4$ und $R_5$ unabhängig voneinander Wasserstoff, Niederalkyl, Niederalkoxy oder Halogen darstellen; wobei die mit "nieder" bezeichneten Reste höchstens 7 Kohlenstoffatome enthalten; oder Tautomere davon, oder Salze davon herstellt.

4. Verfahren nach Anspruch 1, dadurch gekennzeichnet, dass man Verbindungen der Formel III

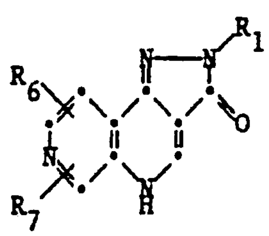 (III),

worin $R_1$ gegebenenfalls durch Niederalkyl, Niederalkoxy, Halogen oder Trifluoromethyl mono- oder disubstituiertes Phenyl bedeutet; oder $R_1$ einen aromatischen heterocyclischen Rest aus der Gruppe Pyridyl, Chinolyl, Isochinolyl, Pyrimidyl und Thiazolyl bedeutet, oder einen der genannten Reste, der durch Niederalkyl, Niederalkoxy oder Halogen mono- oder disubstituiert ist; $R_6$ und $R_7$ unabhängig voneinander Wasserstoff, Niederalkyl, Niederalkoxy oder Halogen bedeuten; wobei die mit "nieder" bezeichneten Reste höchstens 7 Kohlenstoffatome enthalten; oder Tautomere davon; oder Salze davon herstellt.

5. Verfahren nach Anspruch 1, dadurch gekennzeichnet, dass man Verbindungen der im Anspruch 1 gezeigten Formeln IA und IB, worin A zusammen mit den zwei Kohlenstoffatomen, an welche es gebunden ist, ankondensiertes 2,3- oder 3,2-Thieno darstellt, und wobei die Kohlenstoffatome im genannten Thienoring unsubstituiert sind oder einer davon durch Niederalkyl, Carbo(nieder)-alkoxy, Halogen oder Trifluoromethyl substituiert ist, wobei die mit "nieder" bezeichneten Reste höchstens 7 Kohlenstoffatome enthalten, und $R_1$, $R_2$, $R_3$ und $R'_3$ die im Anspruch 1 angegebene Bedeutung haben; oder Salze davon herstellt.

6. Verfahren nach Anspruch 1, dadurch gekennzeichnet, dass man Verbindungen der im Anspruch 1 gezeigten Formeln IA und IB, worin A zusammen mit den zwei Kohlenstoffatomen, an welche es gebunden ist, gegebenenfalls N-Niederalkyl substituiertes ankondensiertes 5,4-Imidazo darstellt, und das Kohlenstoff im genannten 5,4-Imidazoring gegebenenfalls durch Niederalkyl, oder gegebenenfalls durch Niederalkyl, Niederalkoxy, Halogen oder Trifluoromethyl mono-substituiertes Phenyl substituiert ist, wobei die mit "nieder" bezeichneten Reste höchstens 7 Kohlenstoffatome enthalten, und $R_1$, $R_2$, $R_3$ und $R'_3$ die im Anspruch 1 angegebene Bedeutung haben; oder Salze davon herstellt.

7. Verfahren nach Anspruch 1, dadurch gekennzeichnet, dass man 2-Phenyl-pyrazolo[4,3-c][1,6]naphthyridin-3(5H)-on oder seine Salze herstellt.

8. Verfahren nach Anspruch 1, dadurch gekennzeichnet, dass man 2-(3-Pyridyl)-pyrazolo[4,3-c][1,6]napthyridin-3(5H)-on oder seine Salze herstellt.

9. Verfahren nach Anspruch 1, dadurch gekennzeichnet, dass man 2-(2-Thiazolyl)-pyrazolo[4,3-c][1,6]naphthyridin-3(5H)-on oder seine Salze herstellt.

10. Verfahren nach Anspruch 1, dadurch gekennzeichnet, dass man 2-p-Chlorphenyl-pyrazolo[4,3-c][1,7]naphthyridin-3(5H)-on oder seine Salze herstellt.

11. Verfahren zur Herstellung eines pharmazeutischen Präparates, gekennzeichnet durch die Verarbeitung eines erfindungsgemässen Wirkstoffs nach einem der Ansprüche 1 bis 10, mit einem pharmazeutischen Trägermaterial.

**Claims**

for the Contracting States: DE, GB, FR, CH, LI, IT, NL, BE, SE, LU

1. A 2-substituted [b]-heterocycle-fused pyrazolo[3,4-d]pyridin-3-one of the formula IA or IB

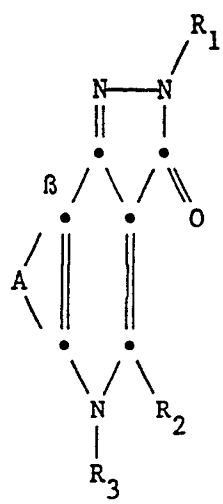
(IA) or
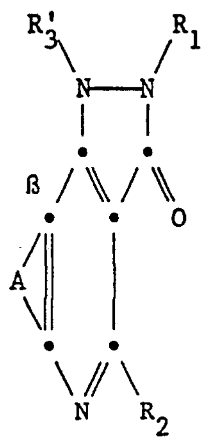
(IB)

wherein A together with the two carbon atoms to which it is attached represents a fused unsaturated heterocyclic ring selected from (a) thieno, furo, and N-unsubstituted or N-lower alkyl substituted pyrrolo, wherein the carbon atoms in any of said rings are unsubstituted or one of them is substituted by lower alkyl, carbo-(lower)-alkoxy, halogen or trifluoromethyl; (b) N-unsubstituted or N-lower alkyl substituted imidazo, wherein the carbon atom in said ring is unsubstituted or substituted by lower alkyl, lower alkoxy, halogen or trifluoromethyl; (c) thiazolo, oxazolo, isoxazolo, 2,3-, 3,4- or 4,3-pyrido, and pyridazino, wherein the carbon atoms forming any of said rings are unsubstituted or one or two are substituted by lower alkyl, lower alkoxy or halogen; (d) pyrimido and pyrazino wherein the carbon atoms in any of said rings are unsubstituted or one is substituted by lower alkyl or lower alkoxy; $R_1$ represents phenyl, or phenyl mono- or disubstituted by lower alkyl, lower alkoxy, halogen or trifluoromethyl; or $R_1$ represents an aromatic heterocyclic radical selected from pyridyl, quinolyl, isoquinolyl, pyrimidyl and thiazolyl, or any of said heterocyclic radicals mono- or di-substituted by lower alkyl, lower alkoxy or halogen; and $R_2$, $R_3$ and $R'_3$ independently represent hydrogen or lower alkyl; with the radicals qualified by the term "lower" containing at most 7 carbon atoms; or a tautomer thereof; or a salt thereof.

2. A compound of formula IA or IB according to claim 1, wherein A together with the two carbon atoms to which it is attached represents fused 2,3-, 3,4- or 4,3-pyrido, or any of said pyrido rings mono- or di-substituted by lower alkyl, lower alkoxy or halogen; with the radicals qualified by the term "lower" containing at most 7 carbon atoms; and $R_1$, $R_2$, $R_3$ and $R'_3$ are as defined in claim 1; or a tautomer thereof; or a pharmaceutically acceptable salt thereof.

3. A compound of formula II according to claim 1

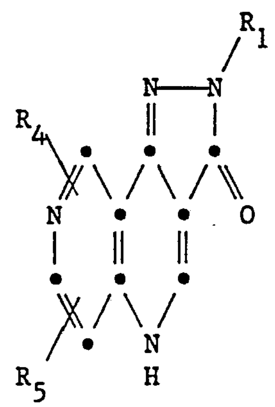

(II)

wherein $R_1$ represents phenyl or phenyl mono- or di-substituted by lower alkyl lower alkoxy, halogen or trifluormethyl; or $R_1$ represents an aromatic heterocyclic radical selected from pyridyl, quinolyl, isoquinolyl, pyrimidyl and thiazolyl, or any said radical mono- or di-substituted by lower alkyl, lower alkoxy or halogen; $R_4$ and $R_5$ represent independently hydrogen, lower alkyl, lower alkoxy or halogen; with the radicals qualified by the term "lower" containing at most 7 carbon atoms; or a tautomer thereof; or a pharmaceutically acceptable salt thereof.

4. A compound of formula III according to claim 1

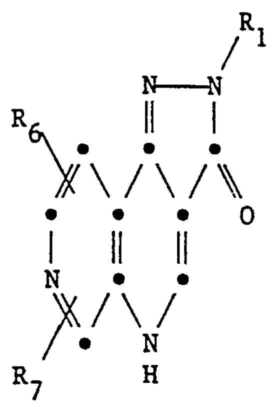

(III)

wherein $R_1$ represents phenyl or phenyl mono- or di-substituted by lower alkyl, lower alkoxy, halogen or trifluoromethyl; or $R_1$ represents an aromatic heterocyclic radical selected from pyridyl, quinolyl, isoquinolyl, pyrimidyl and thiazolyl, or any said radical mono- or di-substituted by lower alkyl, lower alkoxy or halogen; $R_6$ and $R_7$ represent independently hydrogen, lower alkyl, lower alkoxy or halogen; with the radicals qualified by the term "lower" containing at most 7 carbon atoms; or a tautomer thereof; or a pharmaceutically acceptable salt thereof.

5. A compound of formula IA or IB according to claim 1, wherein A together with the two carbon atoms to which it is attached represents fused 2,3- or 3,2-thieno, and the carbon atoms in said thieno ring are unsubstituted or one is substituted by lower alkyl, carbo-(lower)-alkoxy, halogen or trifluoromethyl; with the radicals qualified by the term "lower" containing at most 7 carbon atoms; and $R_1$, $R_2$, $R_3$ and $R'_3$ are as defined in claim 1; or a pharmaceutically acceptable salt thereof.

6. A compound of formula IA or IB according to claim 1, wherein A together with the two carbon atoms to which it is attached represents fused N-unsubstituted or N-lower alkyl substituted 5,4-imidazo, and the carbon atom in said 5,4-imidazo ring is unsubstituted or substituted by lower alkyl, phenyl or phenyl mono-substituted by lower alkyl, lower alkoxy, halogen, or trifluoromethyl; with the radicals qualified by the term "lower" containing at most 7 carbon atoms; and $R_1$, $R_2$, $R_3$ and $R'_3$ are as defined in claim 1; or pharmaceutically acceptable salt thereof.

7. 2-Phenyl-pyrazolo-[4,3-c][1,6]naphthyridin-3(5H)-one or a pharmaceutically acceptable salt thereof.

8. 2-(3-Pyridyl)-pyrazolo[4,3-c][1,6]naphthyridin-3(5H)-one or a pharmaceutically acceptable salt thereof.

9. 2-(2-Thiazolyl)-pyrazolo[4,3-c][1,6]naphthyridin-3(5H)-one or a pharmaceutically acceptable salt thereof.

10. 2-p-Chlorophenyl-pyrazolo[4,3-c][1,7]naphthyridin-3(5H)-one or a phamaceutically acceptale salt thereof.

11. A pharmaceutical preparation comprising a compound as claimed in any one of claims 1 to 10 in admixture or conjunction with a pharmaceutically suitable carrier.

12. A compound as claimed in any one of claims 1 to 10 for use in a therapeutic method of treating the human

or animal body.

13. Use of a compound as claimed in any one of claims 1 to 10 for the preparation of a pharmaceutical agent with benzodiazepine receptor modulating properties.

14. Use of a compound as claimed in any one of claims 1 to 10 for the preparation of pharmaceutical compositions.

15. A process for the preparation of a compound of formula IA or IB as claimed in claim 1, which process comprises

a) reacting a compound of formula VII

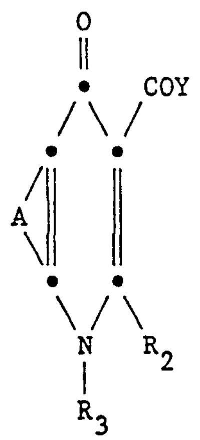

(VII)

wherein A, $R_2$ and $R_3$ are as defined in claim 1, and Y is lower alkoxy; with a compound of the formula VIII

$R'_3$-NH-NH-$R_1$ (VIII)

wherein $R_1$ is as defined in claim 1, and $R'_3$ is hydrogen; or

b) reacting a compound of the formula IX

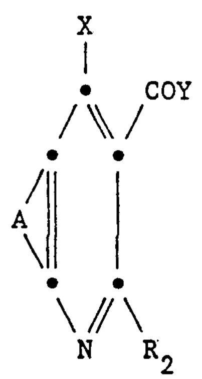

(IX)

wherein A and $R_2$ are as defined in claim 1; X represents reactive etherified or esterified hydroxy; and Y represents lower alkoxy; with a compound of formula VIII wherein $R_1$ is as defined in claim 1; and $R'_3$ represents hydrogen or lower alkyl; or

c) ring closing a compound of formula IX wherein X is -$NR'_3$-$NHR_1$ and Y is lower alkoxy or hydroxy; or X is hydroxy, reactive esterified or etherified hydroxy and Y is -$NR_1NHR'_3$; and wherein A, $R_1$, $R_2$ and $R'_3$ are as defined in claim 1; or

d) ring-closing a compound of formula IX wherein X is lower alkoxyamino or azido, and Y is -NH-$R_1$, and A, $R_1$ and $R_2$ are as defined in claim 1; or

e) condensing a compound of formula X

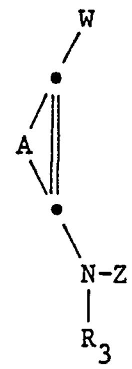

(X)

wherein W and $R_3$ are hydrogen, Z is

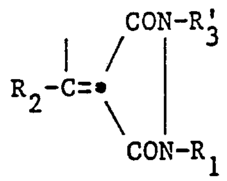

and A, $R_1$ $R_2$ and $R'_3$ are as defined in claim 1; or
f) condensing a compound of formula X wherein W is

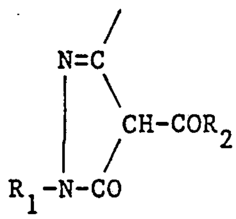

and Z is hydrogen, and A, $R_1$, $R_2$ and $R_3$ are as defined in claim 1; or
g) condensing a compound of formula X wherein W is

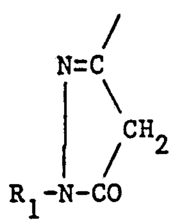

,

Z is $R_2CO$- or 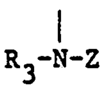

is isocyano, and A, $R_1$, $R_2$ and $R_3$ are as defined in claim 1; if desired, while temporarily protecting any interfering reactive group in all these processes, and isolating the resultant compound of formula IA or IB; and, if desired, converting a resultant compound of formula IA or IB into another compound of the invention, and/or, if desired, converting a resultant free compound into a salt or a resultant salt into the free compound or into another salt, and/or, if desired, resolving a mixture of isomers or racemates obtained into the single isomers or racemates, and/or, if desired, resolving a racemate obtained into the optical antipodes.

16. A compound obtainable by the process accordimg to claim 15.

**Claims**

for the Contracting State: AT

1. A process for the preparation of a novel 2-substituted [b]-heterocycle-fused pyrazolo[3,4-d]pyridin-3-one of formula IA or IB

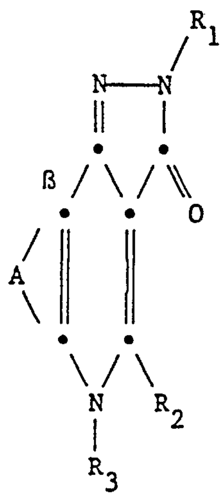 (IA) or 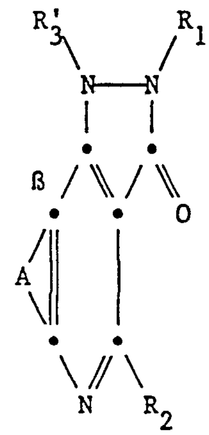 (IB)

wherein A together with the two carbon atoms to which it is attached represents a fused unsaturated heterocyclic ring selected from (a) thieno, furo, and N-unsubstituted or N-lower alkyl substituted pyrrolo, wherein the carbon atoms in any of said rings are unsubstituted or one of them is substituted by lower alkyl, carbo-(lower)-alkoxy, halogen or trifluoromethyl; (b) N-unsubstituted or N-lower alkyl substituted imidazo, wherein the carbon atom in said ring is unsubstituted or substituted by lower alkyl, lower alkoxy, halogen or trifluoromethyl; (c) thiazolo, oxazolo, isoxazolo, 2,3-, 3,4- or 4,3-pyrido, and pyridazino, wherein the carbon atoms forming any of said rings are unsubstituted or one or two are substituted by lower alkyl, lower alkoxy or halogen; (d) pyrimido and pyrazino wherein the carbon atoms in any of said rings are unsubstituted or one is substituted by lower alkyl or lower alkoxy; $R_1$ represents phenyl, or phenyl mono- or di-substituted by lower alkyl, lower alkoxy, halogen or trifluoromethyl; or $R_1$ represents an aromatic heterocyclic radical selected from pyridyl, quinolyl, isoquinolyl, pyrimidyl and thiazolyl, or any of said heterocyclic radicals mono- or di-substituted by lower alkyl, lower alkoxy or halogen; and $R_2$, $R_3$ and $R'_3$ independently represent hydrogen or lower alkyl; with the radicals qualified by the term "lower" containing at most 7 carbon atoms; or of a tautomer thereof; or of a salt thereof; which process comprises

a) reacting a compound of formula VII

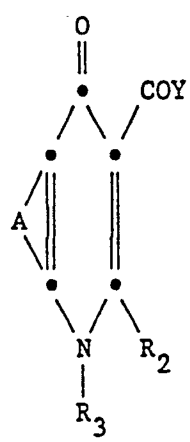

(VII)

wherein A, $R_2$ and $R_3$ are as defined above, and Y is lower alkoxy; with a compound of the formula VIII

$R'_3$-NH-NH-$R_1$ (VIII)

wherein $R_1$ is as defined above, and $R'_3$ is hydrogen; or

b) reacting a compound of the formula IX

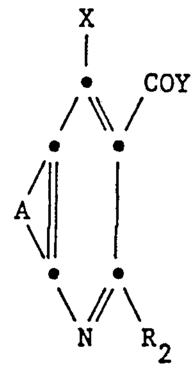

(IX)

wherein A and $R_2$ are as defined above; X represents reactive etherified or esterified hydroxy; and Y represents lower alkoxy; with a compound of formula VIII wherein $R_1$ is as defined above; and $R'_3$ represents hydrogen or lower alkyl; or

c) ring closing a compound of formula IX wherein X is $-NR'_3-NHR_1$ and Y is lower alkoxy or hydroxy; or X is hydroxy, reactive esterified or etherified hydroxy and Y is $-NR_1NHR'_3$; and wherein A, $R_1$, $R_2$ and $R'_3$ are as defined above; or

d) ring-closing a compound of formula IX wherein X is lower alkoxyamino or azido, and Y is $-NH-R_1$, and A, $R_1$ and $R_2$ are as defined above; or

e) condensing a compound of formula X

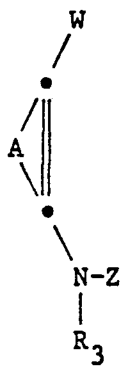

(X)

wherein W and $R_3$ are hydrogen, Z is

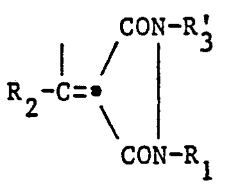

and A, $R_1$, $R_2$ and $R'_3$ are as defined above; or

f) condensing a compound of formula X wherein W is

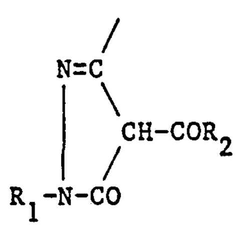

and Z is hydrogen, and A, $R_1$, $R_2$ and $R_3$ are as defined above; or

g) condensing a compound of formula X wherein W is

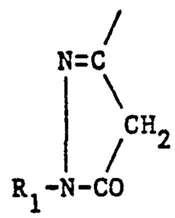 ,

Z is $R_2CO$- or 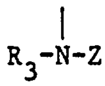

is isocyano, and A, $R_1$, $R_2$, $R_3$ as defined above; if desired, while temporarily protecting any interfering reactive group in all these processes, and isolating the resultant compound of formula IA or IB; and, if desired, converting a resultant compound of formula IA or IB into another compound of the invention, and/or, if desired, converting a resultant free compound into a salt or a resultant salt into the free compound or into another salt, and/or, if desired, resolving a mixture of isomers or racemates obtained into the single isomers or racemates, and/or, if desired, resolving a racemate obtained into the optical antipodes.

2. A process according to claim 1, which comprises preparing a compound of the formula IA or IB indicated in claim 1, wherein A together with the two carbon atoms to which it is attached represents fused 2,3-, 3,4- or 4,3-pyrido, or any of said pyrido rings mono- or di-substituted by lower alkyl, lower alkoxy or halogen; with the radicals qualified by the term "lower" containing at most 7 carbon atoms; and $R_1$, $R_2$, $R_3$ and $R'_3$ are as defined in claim 1; or a salt thereof.

3. A process according to claim 1, which comprises preparing a compound of formula II

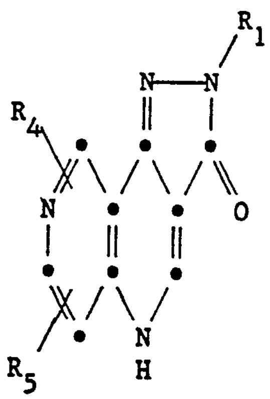

(II)

wherein $R_1$ represents phenyl or phenyl mono- or di-substituted by lower alkyl, lower alkoxy, halogen or trifluoromethyl; or $R_1$ represents an aromatic heterocyclic radical selected from pyridyl, quinolyl, isoquinolyl, pyrimidyl and thiazolyl, or any said radical mono- or disubstituted by lower alkyl, lower alkoxy or halogen; $R_4$ and $R_5$ represent independently hydrogen, lower alkyl, lower alkoxy or halogen; with the radicals qualified by the term "lower" containing at most 7 carbon atoms; or a tautomer thereof; or a salt thereof.

4. A process according to claim 1, which comprises preparing a compound of formula III

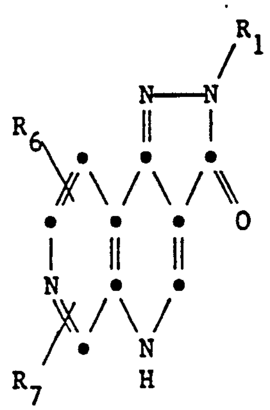

(III)

wherein $R_1$ represents phenyl or phenyl mono- or di-substituted by lower alkyl, lower alkoxy, halogen or trifluoromethyl; or $R_1$ represents an aromatic heterocyclic radical selected from pyridyl, quinolyl, isoquinolyl,

pyrimidyl and thiazolyl, or any said radical mono- or di-substituted by lower alkyl, lower alkoxy or halogen; $R_6$ and $R_7$ represent independently hydrogen, lower alkyl, lower alkoxy or halogen; with the radicals qualified by the term "lower" containing at most 7 carbon atoms; or a tautomer thereof; or a salt thereof.

5. A process according to claim 1, which comprises preparing a compound of the formula IA or IB indicated in claim 1, wherein A together with the two carbon atoms to which it is attached represents fused 2,3- or 3,2-thieno, and the carbon atoms in said thieno ring are unsubstituted or one is substituted by lower alkyl, carbo-(lower)-alkoxy, halogen or trifluoromethyl; with the radicals qualified by the term "lower" containing at most 7 carbon atoms; and $R_1$, $R_2$, $R_3$ and $R'_3$ are as defined in claim 1; or a salt thereof.

6. A process according to claim 1, which comprises preparing a compound of the formula IA or IB indicated in claim 1, wherein A together with the two carbon atoms to which it is attached represents fused N-unsubstituted or N-lower alkyl substituted 5,4-imidazo, and the carbon atom in said 5,4-imidazo ring is unsubstituted or substituted by lower alkyl, phenyl or phenyl mono-substituted by lower alkyl, lower alkoxy, halogen, or trifluoromethyl; with the radicals qualified by the term "lower" containing at most 7 carbon atoms; and $R_1$, $R_2$, $R_3$ and $R'_3$ are as defined in claim 1; or a salt thereof.

7. A process according to claim 1, which comprises preparing 2-phenyl-pyrazolo-[4,3-c][1,6]naphthyridin-3(5H)-one or a salt thereof.

8. A process according to claim 1, which comprises preparing 2-(3-pyridyl)-pyrazolo[4,3-c][1,6]naphthyridin-3(5H)-one or a salt thereof.

9. A process according to claim 1, which comprises preparing 2-(2-thiazolyl)-pyrazolo[4,3-c][1,6]naphthyridin-3(5H)-one or a salt thereof.

10. A process according to claim 1, which comprises preparing 2-p-chlorophenyl-pyrazolo[4,3-c][1,7]naphthyridin-3(5H)-one or a salt thereof.

11. A process for the preparation of a pharmaceutical composition, which process comprises combining a compound obtained in accordance with any one of claims 1 to 10 with a pharmaceutically suitable carrier.

**Revendications**

pour les Etats contractants: DE, GB, FR, CH, LI, IT, NL, BE, SE, LU

1 - Pyrazolo[3,4-d]-pyridine-3-ones condensées avec un noyau hétérocyclique en [b] et substituées en position 2, répondant aux formules IA ou IB

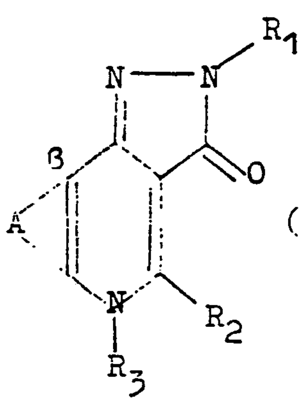

(IA) ou

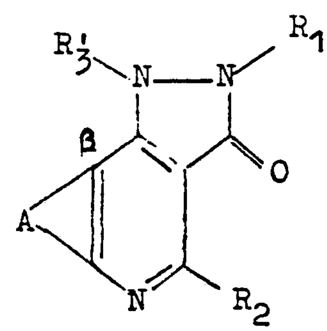

(IB)

dans lesquelles A forme avec les deux atomes de carbone auxquels il est relié un noyau hétérocyclique insaturé condensé du groupe (a) thiéno, furo et pyrrolo éventuellement substitué par un groupe alkyle inférieur sur l'azote, les atomes de carbone de chacun de ces noyaux étant non substitués ou bien l'un d'entre eux étant substitué par un groupe alkyle inférieur, carboalcoxy inférieur, un halogène ou un groupe trifluorométhyle; (b) imidazo éventuellement substitué à l'azote par un groupe alkyle inférieur, l'atome de carbone du noyau en question étant éventuellement substitué par un groupe alkyle inférieur, alcoxy inférieur, un halogène ou un groupe trifluorométhyle; (c) thiazolo, oxazolo, isoxazolo, 2,3-, 3,4- ou 4,3-pyrido et pyridazino, les atomes de carbone formant ces noyaux étant non substitués ou bien un ou deux d'entre eux étant substitués par des groupes alkyle inférieurs, alcoxy inférieurs ou un halogène; (d) pyrimido et pyrazino, les atomes de carbone de ces noyaux étant non substitués ou bien l'un d'entre eux étant substitué par un groupe alkyle inférieur ou alcoxy inférieur; $R_1$ représente un groupe phényle éventuellement mono- ou di-substitué par des groupes alkyle inférieurs, alcoxy inférieurs, des halogènes ou des groupes trifluorométhyle; ou bien $R_1$ représente un radical hétérocyclique aromatique du groupe pyridyle, quinoléyle, isoquinoléyle, pyrimidyle et thiazolyle ou l'un quelconque des radicaux hétérocycliques mentionnés, qui sont mono- ou di-substitués par des groupes alkyle inférieurs, alcoxy inférieurs ou des halogènes; et $R_2$, $R_3$ et $R'_3$ représentent chacun, indépendamment les uns des autres, l'hydrogène ou un groupe alkyle inférieur; les groupes qualifiés d'"inférieurs" contenant au maximum 7 atomes de carbone, leurs tautomères ou leurs sels.

2 - Composés de formules IA ou IB selon la revendication 1, dans lesquels A forme avec les deux atomes de carbone auxquels il est relié des noyaux 2,3-, 3,4-ou 4,3-pyrido condensés ou l'un quelconque des noyaux pyrido mentionnés, qui sont mono- ou di-substitués par des groupes alkyle inférieurs, alcoxy inférieurs ou des halogènes; les groupes qualifiés d'"inférieura" contenant au maximum 7 atomes de carbone, et $R_1$, $R_2$, $R_3$ et $R'_3$ ont les significations indiquées dans la revendication 1; ou leurs tautomères ou sels acceptables pour l'usage pharmaceutique.

3 - Composés de formule II selon la revendication 1

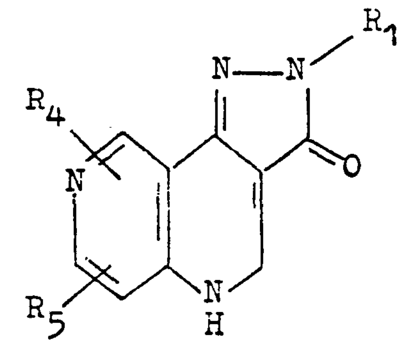

(II),

dans laquelle $R_1$ représente un groupe phényle mono- ou di-substitué par des groupes alkyle inférieurs, alcoxy inférieurs, des halogènes ou des groupes trifluorométhyle; ou bien $R_1$ représente un noyau hétérocyclique aromatique du groupe pyridyle, quinoléyle, isoquinoléyle, pyrimidyle ou thiazolyle, ou bien l'un de ces radicaux, qui est mono- ou di-substitué par des groupes alkyle inférieurs, alcoxy inférieur ou des halogènes; $R_4$ et $R_5$ représentent chacun, indépendamment l'un de l'autre, l'hydrogène, un groupe alkyle inférieur, alcoxy inférieur ou un halogène; les groupes qualifiés d'"inférieurs" contenant au maximum 7 atomes de carbone, ou leurs tautomères ou leurs sels acceptables pour l'usage pharmaceutique.

4 - Composés de formule III selon la revendication 1

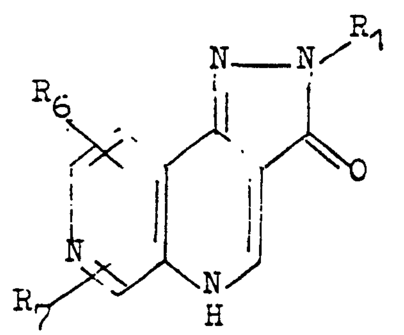

(III),

dans laquelle $R_1$ représente un groupe phényle mono- ou di-substitué par des groupes alkyle inférieurs, alcoxy inférieurs, des halogènes ou des groupes trifluorométhyle ou bien $R_1$ représente un noyau hétérocyclique aromatique du groupe pyridyle, quinoléyle, isoquinoléyle, pyrimidyle et thiazolyle, ou l'un des radicaux mentionnés, qui est mono- ou di-substitué par des groupes alkyle inférieurs, alcoxy inférieurs ou des halogènes; $R_6$ et $R_7$ représentent chacun, indépendamment l'un de l'autre, l'hydrogène, un groupe alkyle inférieur, alcoxy inférieur ou un halogène; les groupes qualifiés d'"inférieurs" contenant au maximum 7 atomes de carbone, ou leurs tautomères; ou leurs sels acceptables pour l'usage pharmaceutique.

5 - Composés de formules IA ou IB selon la revendication 1, dans lesquels A forme avec les deux atomes de carbone auxquels il est relié un noyau 2,3- ou 3,2-thiéno condensé, les atomes de carbone de ce noyau thiéno étant non substitués ou bien l'un d'entre eux étant substitué par un groupe alkyle inférieur, carbalcoxy inférieur, un halogène ou un groupe trifluorométhyle; les groupes qualifiés d'"inférieurs" contenant au maximum 7 atomes de carbone, et $R_1$, $R_2$, $R_3$ et $R_3'$ ont les significations indiquées dans la revendication 1; ou leurs sels acceptables pour l'usage pharmaceutique.

6 - Composés de formules IA ou IB selon la revendication 1, dans lesquels A forme avec les deux atomes de carbone auxquels il est relié un noyau 5,4-imidazo condensé éventuellement substitué à l'azote par un groupe alkyle inférieur, et l'atome de carbone de ce noyau 5,4-imidazo est éventuellement substitué par un groupe alkyle inférieur ou par un groupe phényle éventuellement monosubstitué par un groupe alkyle inférieur, alcoxy inférieur, un halogène ou un groupe trifluorométhyle; les groupes qualifiés d'"inférieurs" contenant au maximum 7 atomes de carbone, et $R_1$, $R_2$, $R_3$ et $R'_3$ ont les significations indiquées dans la revendication 1; ou leurs sels acceptables pour l'usage pharmaceutique.

7 - La 2-phényl-pyrazolo-[4,3-c][1,6]naphtiridine-3(5H)-one ou ses sels acceptables pour l'usage pharmaceutique.

8 - La 2-(3-pyridyl)-pyrazolo[4,3-c][1,6]naphtiridine-3(5H)-one ou ses sels acceptables pour l'usage pharmaceutique.

9 - La 2-(2-thiazolyl)-pyrazolo[4,3-c][1,6]-naphtiridine-3(5H)-one ou ses sels acceptables pour l'usage pharmaceutique.

10 - La 2-p-chlorophényl-pyrazolo[4,3-c][1,7]-naphtiridine-3(5H)-one ou ses sels acceptables pour l'usage pharmaceutique.

11 - Compositions pharmaceutiques contenant un composé des revendications 1 à 10, avec un véhicule pharmaceutique.

12 - Les composés mentionnés dans les revendications 1 à 10, pour l'utilisation dans un procédé pour le traitement thérapeutique de l'organisme humain ou animal.

13 - Utilisation des composés mentionnés dans les revendications 1 à 10 pour la préparation d'un médicament possédant des propriétés de modulation des récepteurs des benzodiazépines.

14 - Utilisation des composés mentionnés dans les revendications 1 à 10, pour la préparation de compositions pharmaceutiques.

15 - Procédé de préparation des composés de formules IA ou IB mentionnés dans la revendication 1, caractérisé en ce que:

a) on fait réagir un composé de formule VII

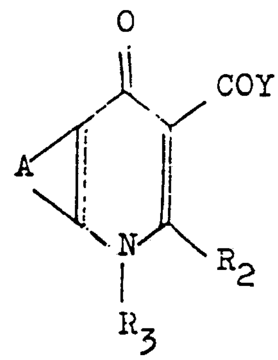

(VII),

dans laquelle A, $R_2$ et $R_3$ ont les significations indiquées dans la revendication 1, et Y représente un groupe alcoxy inférieur, avec un composé de formule VIII

$R'_3$-NH-NH-$R_1$ (VIII)

dans laquelle $R_1$ a les significations indiquées dans la revendication 1, et $R'_3$ représente l'hydrogène, ou bien

b) on fait réagir un composé de formule IX

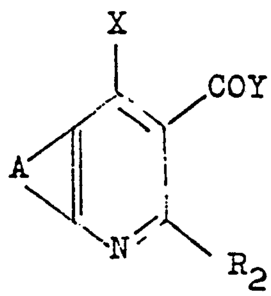

(IX),

dans laquelle A et $R_2$ ont les significations indiquées dans la revendication 1, X représente un groupe hydroxy éthérifié ou estérifié réactif et Y représente un groupe alcoxy inférieur, avec un composé de formule VIII dans laquelle $R_1$ a les significations indiquées dans la revendication 1 et $R'_3$ représente l'hydrogène ou un groupe alkyle inférieur, ou bien

c) on cyclise un composé de formule IX dans laquelle X représente le groupe -$NR'_3$-$NHR_1$, Y représente un groupe alcoxy inférieur ou hydroxy, ou bien X représente un groupe hydroxy, un groupe hydroxy estérifié ou étherifié réactif, et Y représente le groupe -$NR_1$-$NHR'_3$; et A, $R_1$, $R_2$ et $R'_3$ ont les significations indiquées dans la revendication 1; ou bien

d) on cyclise un composé de formule IX dans laquelle X représente un groupe alcoxy inférieur, amino ou azido et Y représente un groupe -NH-$R_1$, A, $R_1$ et $R_2$ ayant les significations indiquées dans la revendication 1; ou bien

e) on condense un composé de formule X

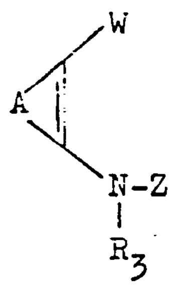

(X),

dans laquelle W et $R_3$ représentent l'hydrogène, Z représente

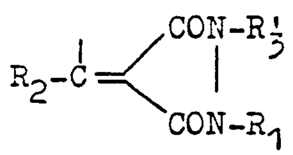

et A, $R_1$, $R_2$ et $R'_3$ ont les significations indiquées dans la revendication 1; ou bien

f) on condense un composé de formule X dans laquelle W représente

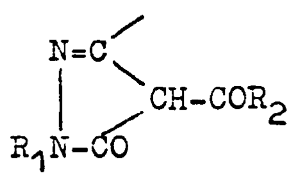

et Z représente l'hydrogène, et A, $R_1$, $R_2$ et $R_3$ ont les significations indiquées dans la revendication 1; ou bien

g) on condense un composé de formule X dans laquelle W représente

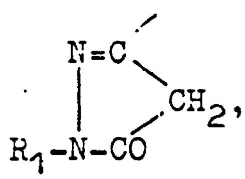

et Z représente $R_2CO$- ou 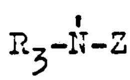

représente un groupe isocyano, et A, $R_1$, $R_2$ et $R_3$ ont les significations indiquées dans la revendication 1, si on le désire, dans tous ces procédés, on protège transitoirement un groupe réactif gênant, et on isole le composé obtenu répondant à la formule IA ou IB; et, si on le désire, on convertit un composé obtenu répondant à la formule IA ou IB en un autre composé selon l'invention, et/ou, si on le désire, on convertit un composé obtenu en un sel ou un sel obtenu en le composé libre ou en un autre sel, et/ou, si on le désire, on sépare un mélange d'isomères ou de racémates obtenu en les isomères ou racémates individuels et/ou, si on le désire, on sépare les racémates obtenus en les antipodes optiques.

16 - Les composés qu'on peut obtenir par le procédé de la revendication 15.

**Revendications**

pour l'Etat Contractant: AT

1 - Procédé de préparation des nouvelles pyrazolo[3,4-d]-pyridine-3-ones condensées avec un noyau hétérocyclique en [b] et substituées en position 2, répondant aux formules IA ou IB

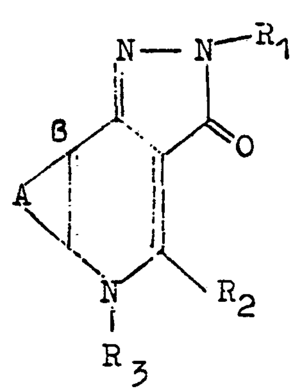

(IA) ou

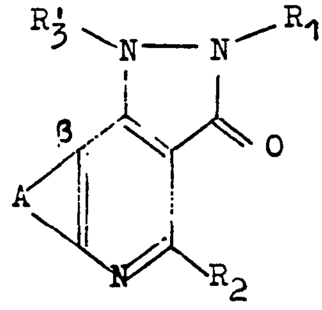

(IB)

dans lesquelles A forme avec les deux atomes de carbone auxquels il est relié un noyau hétérocyclique insaturé condensé du groupe (a) thiéno, furo et pyrrolo éventuellement substitué par un groupe alkyle inférieur à l'azote, les atomes de carbone de chacun de cex noyaux étant non substitués ou bien l'un d'entre eux étant substitué par un groupe alkyle inférieur, carbo-(alcoxy inférieur), un halogène ou un groupe trifluorométhyle; (b) imidazo éventuellement substitué par un groupe alkyle inférieur à l'azote, l'atome de carbone du noyau mentionné étant éventuellement substitué par un groupe alkyle inférieur, alcoxy inférieur, un halogène ou un groupe trifluorométhyle; (c) thiazolo, oxazolo, isoxazolo, 2,3-, 3,4- ou 4,3-pyrido et pyridazino, les atomes de carbone formant ces noyaux étant non substitués ou bien un ou deux d'entre eux étant substitués par des groupes alkyle inférieurs, alcoxy inférieurs ou des halogènes; (d) pyrimido et pyrazino, les atomes de carbone de ces noyaux étant non substitués ou bien l'un d'entre eux étant substitué par un groupe alkyle inférieur ou alcoxy inférieur; $R_1$ représente un groupe phényle éventuellement mono- ou di-substitué par des groupes alkyle inférieurs, alcoxy inférieurs, des halogènes ou des groupes trifluorométhyle; ou bien $R_1$ représente un noyau hétérocyclique aromatique du groupe pyridyle, quinoléyle, isoquinoléyle, pyrimidyle et thiazolyle ou représente l'un quelconque des noyaux hétérocycliques mentionnés qui est mono- ou di-substitué par des groupes alkyle inférieurs, alcoxy inférieurs ou des halogènes; et $R_2$, $R_3$ et $R'_3$ représentent chacun, indépendamment l'un de l'autre, l'hydrogène ou un groupe alkyle inférieur, les groupes qualifiés d'"inférieurs" contenant au maximum 7 atomes de carbone, ou de leurs tautomères ou de leurs sels, caractérisé en ce que

a) on fait réagir un composé de formule VII

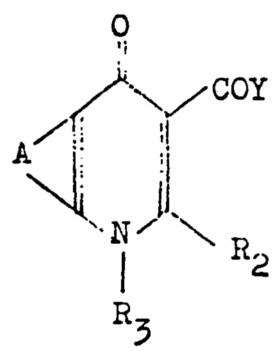

(VII),

dans laquelle A, $R_2$ et $R_3$ ont les significations indiquées ci-dessus et Y représente un groupe alcoxy inférieur, avec un composé de formule VIII

$R'_3$-NH-NH-$R_1$ (VIII),

dans laquelle $R_1$ a les significations indiquées ci-dessus et $R'_3$ représente l'hydrogène, ou bien

b) on fait réagir un composé de formule IX

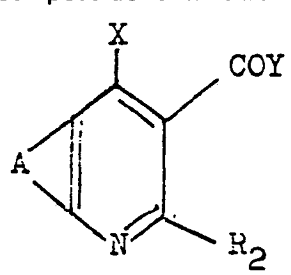

(IX),

dans laquelle A et $R_2$ ont les significations indiquées ci-dessus, X représente un groupe hydroxy éthérifié ou estérifié réactif et Y un groupe alcoxy inférieur, avec un compose de formule VIII dans laquelle $R_1$ a les significations indiquées ci-dessus et $R'_3$ représente l'hydrogène ou un groupe alkyle inférieur; ou bien

c) on cyclise un composé de formule IX dans laquelle X représente le groupe -$NR'_3$-$NHR_1$, Y représente un

groupe alcoxy inférieur ou hydroxy, ou bien X représente un groupe hydroxy, un groupe hydroxy éthérifié ou estérifié réactif et Y le groupe $-NR_1-NHR_3$, A, $R_1$, $R_2$ et $R_3$ ayant les significations indiquées ci-dessus; ou bien

d) on cyclise un composé de formule IX dans laquelle X représente un groupe alcoxy inférieur, amino ou azido et Y représente le groupe $-NH-R_1$, A, $R_1$ et $R_2$ ayant les significations indiquées ci-dessus; ou bien

e) on condense un composé de formule X

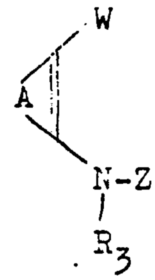

(X)

dans laquelle W et $R_3$ représentent l'hydrogène, Z représente

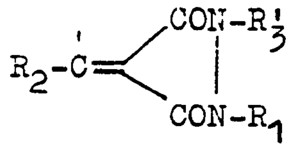

et A, $R_1$, $R_2$ et $R'_3$ ont les significations indiquées ci-dessus; ou bien

f) on condense un composé de formule X dans laquelle W représente

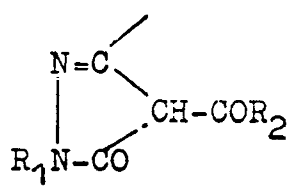

et Z représente l'hydrogène, A, $R_1$, $R_2$ et $R_3$ ayant les significations indiquées ci-dessus; ou bien

g) on condense un composé de formule X dans laquelle W représente

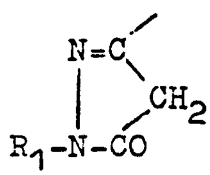

,

et Z représente le groupe $R_2CO-$ ou bien $R_3-N-Z$

représente un groupe isocyano, et A, $R_1$, $R_2$ et $R_3$ ont les significations indiquées ci-dessus, si on le désire, dans tous ces procédés, on protège transitoirement un groupe réactif gênant et on isole le composé obtenu répondant à la formule IA ou IB; et si on le désire, on convertit un composé obtenu de formule IA ou IB en un autre composé selon l'invention, et/ou si on le désire, on convertit un composé obtenu en un sel ou un sel obtenu en le composé libre ou en un autre sel, et/ou si on le désire, on sépare un mélange d'isomères ou de racémates obtenu en les isomères ou racémates individuels, et/ou si on le désire, on sépare des racémates obtenus en les antipodes optiques.

2 - Procédé selon la revendication 1, caractérisé en ce que l'on prépare des composés de formules IA et IB de la revendication 1, dans lesquels A forme avec les deux atomes de carbone auxquels il est relié, un noyau 2,3-, 3,4- ou 4,3-pyrido condensé ou l'un quelconque de ces noyaux pyrido mono- ou di-substitués par des groupes alkyle inférieurs, alcoxy inférieurs ou des halogènes; les groupes qualifiés d'"inférieurs" contenant au maximum 7 atomes de carbone, et $R_1$, $R_2$ et $R_3$ et $R'_3$ ont les significations indiquées dans la revendication 1; ou des sels de ces composés.

3 - Procédé selon la revendication 1, caractérisé en ce que l'on prépare des composés de formule II

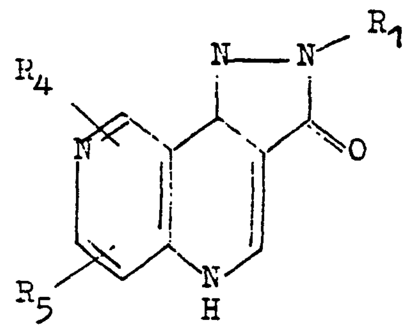

(II),

dans laquelle $R_1$ représente un groupe phényle éventuellement mono- ou di-substitué par des groupes alkyle inférieurs, alcoxy inférieurs, des halogènes ou des groupes trifluorométhyle; ou bien $R_1$ représente un noyau hétérocyclique aromatique du groupe pyridyle, quinoléyle, isoquinoléyle, pyrimidyle ou thiazolyle, ou bien l'un de ces noyaux mono- ou di-substitué par des groupes alkyle inférieurs, alcoxy inférieurs ou des halogènes; $R_4$ et $R_5$ représentent chacun, indépendamment l'un de l'autre, l'hydrogène, un groupe alkyle inférieur, alcoxy inférieur ou un halogène; les groupes qualifiés d'"inférieurs" contenant au maximum 7 atomes de carbone; ou leurs tautomères ou leurs sels.

4 - Procédé selon la revendication 1, caractérisé en ce que l'on prépare des composés de formule III

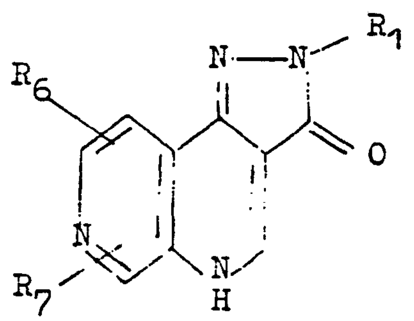

(III),

dans laquelle $R_1$ représente un groupe phényle éventuellement mono- ou di-substitué par des groupes alkyle inférieurs, alcoxy inférieurs, des halogènes ou des groupes trifluorométhyle; ou bien $R_1$ représente un noyau hétérocyclique aromatique du groupe pyridyle, quinoléyle, isoquinoléyle, pyrimidyle et thiazolyle, ou bien l'un de ces noyaux qui est mono- ou di-substitué par des groupes alkyle inférieurs, alcoxy inférieurs ou des halogènes; $R_6$ et $R_7$ représentent chacun, indépendamment l'un de l'autre, l'hydrogène, un groupe alkyle inférieur, alcoxy inférieur ou un halogène; les groupes qualifiés d'"inférieurs" contenant au maximum 7 atomes de carbone, ou leurs tautomères; ou leurs sels.

5 - Procédé selon la revendication 1, caractérisé en ce que l'on prépare des composés de formules IA et IB de la revendication 1, dans lesquels A forme avec les deux atomes de carbone auxquels il est relié un novau 2,3- ou 3,2-thiéno condensé, les atomes de carbone de ce noyau thiéno étant non substitués ou bien l'un d'entre eux étant substitué par un groupe alkyle inférieur, carbo-(alcoxy inférieur), un halogène ou un groupe trifluorométhyle, les groupes qualifiés d'"inférieurs" contenant au maximum 7 atomes de carbone, et $R_1$, $R_2$, $R_3$ et $R'_3$ ayant les significations indiquées dans la revendication 1; ou leurs sels.

6 - Procédé selon la revendication 1, caractérisé en ce que l'on prépare des composés de formules IA et IB de la revendication 1, dans lesquels A forme avec les deux atomes de carbone auxquels il est relié un noyau 5,4-imidazo condensé et éventuellement substitué à l'azote par un groupe alkyle inférieur, et l'atome de carbone de ce noyau 5,4-imidazo est éventuellenent substitué par un groupe alkyle inférieur, ou par un groupe phényle éventuellement monosubstitué par un groupe alkyle inférieur, alcoxy inférieur, un halogène ou un groupe trifluorométhyle, les groupes qualifiés d'"inférieurs" contenant au maximum 7 atomes de carbone, et $R_1$, $R_2$, $R_3$ et $R'_3$ ayant les significations indiquées dans la revendication 1; ou leurs sels.

7 - Procédé selon la revendication 1, caractérisé en ce que l'on prépare la 2-phényl-pyrazolo[4,3-c]-[1,6]naphtiridine-3(5H)-one ou ses sels.

8 - Procédé selon la revendication 1, caractérisé en ce que l'on prépare la 2-(3-pyridyl)-pyrazolo-[4,3-c][1,6]naphtiridine-3(5H)-one ou ses sels.

9 - Procédé selon la revendication 1, caractérisé en ce que l'on prépare la 2-(2-thiazolyl)-pyrazolo-[4,3-c][1,6]naphtiridine-3(5H)-one ou ses sels.

10 - Procédé selon la revendication 1, caractérisé en ce que l'on prépare la 2-p-chlorophényl-pyrazolo-[4,3-c][1,7]naphtiridine-3(5H)-one ou ses sels.

11 - Procédé de préparation d'une composition pharmaceutique, caractérisé en ce que l'on combine une

substance active selon l'invention, selon l'une des revendications 1 à 10, avec un véhicule pharmaceutique.